(19) Europäisches Patentamt
European Patent Office
Office européen des brevets

(11) Publication number: **0 346 702**
**A2**

# (12) EUROPEAN PATENT APPLICATION

(21) Application number: 89109980.6

(22) Date of filing: 02.06.89

(51) Int. Cl.4: C12N 5/00 , C12N 15/00 , C07K 15/06 , //G01N33/574

(30) Priority: 16.06.88 US 207576
27.09.88 US 249922

(43) Date of publication of application:
20.12.89 Bulletin 89/51

(84) Designated Contracting States:
DE FR GB

(71) Applicant: MOLECULAR DIAGNOSTICS, INC.
400 Morgan Lane
West Haven, CT 06516(US)

(72) Inventor: Barnett, Thomas R.
27 Jeffrey Road
East Haven, CT 06513(US)
Inventor: Elting, James J.
5 Heatherwood Drive
Madison, CT 06443(US)
Inventor: Kamarck, Michael E.
86 Russel Road
Bethany, CT 06525(US)
Inventor: Kretschmer, Axel
Richard-Zoerner-Strasse 32
D-5060 Bergisch Gladbach 2(DE)

(74) Representative: Dänner, Klaus, Dr. et al
c/o Bayer AG Konzernverwaltung RP Patente Konzern
D-5090 Leverkusen 1 Bayerwerk(DE)

## (54) Transfectant cell lines which express isolated genes of the carcinoembryonic antigen family.

(57) Non-human mammalian cell lines which express antigens of the carcinoembryonic antigen gene family are provided by the present invention. The cell lines are prepared by gene transfection of human DNA, such as LoVo cell DNA, into non-human mammalian cells, such as mouse cells or by the transfection of complete cDNA in appropriate expression vectors into non-human mammalian cells, such as mouse cells. Primary transfectants can be transfected further into non-human mammalian cells to provide secondary transfectants. Cloning of secondary transfectants yields cell lines that exhibit increased expression of the surface antigen protein. The transfectant cell lines provide a means for producing antibodies, particularly monoclonal antibodies, against CEA family antigens. Furthermore, a general method is provided for screening and assessing the specificity of these antibodies to each member of the CEA gene family.

EP 0 346 702 A2

# Transfectant cell lines which express isolated genes of the carcinoembryonic antigen family

## BACKGROUND OF THE INVENTION

### Field of the Invention

The present invention concerns cell lines which express isolated genes of the carcinoembryonic antigen (CEA) family. The invention also concerns a method for screening antibodies for their specificity to CEA family members using such cell lines. The present invention further relates to a method for generating antibodies specific for specific CEA family members using the aforesaid cell lines.

### Background Information

Carcinoembryonic antigen was first described by Gold and Freedman, J. Exp. Med., 121, 439-462, (1965). CEA is characterized as a glycoprotein of approximately 200,000 molecular weight with 50-60% by weight of carbohydrate. CEA is present during normal human fetal development, but only in very low concentration in the normal adult intestinal tract. It is produced and secreted by a number of different tumors.

CEA is a clinically useful tumor marker for the management of colorectal cancer patients. CEA can be measured using sensitive immunoassay methods. When presurgical serum levels of CEA are elevated, a postsurgical drop in serum CEA to the normal range typically indicates successful resection of the tumor. Postsurgical CEA levels that do not return to normal often indicate incomplete resection of the tumor or the presence of additional tumor sites in the patient. After returning to normal levels, subsequent rapid rises in serum CEA levels usually indicate the presence of metastases. Slower postsurgical rises from the normal level are most often interpreted to indicate the presence of new primary tumors not previously detected. Post surgical management of colon cancer patients is thus facilitated by the measurement of CEA.

CEA is a member of an antigen family. Because of this, the immunoassay of CEA by presently available methods is complicated by the fact that CEA is but one of several potentially reactive antigens. There have been at least sixteen CEA-like antigens described in the literature. Since some of these appear to be the same antigen described by different investigators, the actual number of different antigens is somewhat less than this number. Nonetheless, there is a complex array of cross-reactive antigens which can potentially interfere with an immunoassay of the CEA released by tumors. It is known that serum levels of CEA-like antigens are elevated in many non-cancerous conditions such an inflammatory liver diseases and also in smokers. It is important that immunoassays used for the monitoring of cancer patient status not be interfered with by these other CEA-like antigens. Conversely, it is important to be able to distinguish the antigens by immunoassays because of the possibility that different tumor types may preferentially express different forms of CEA. If so, then the ability to reliably measure the different forms of CEA can provide the means to diagnose or more successfully treat different forms of cancer.

The members of the "CEA family" share some antigenic determinants. These common epitopes are not useful in distinguishing the members of the antigen family and antibodies recognizing them are of little use for measuring tumor-specific CEA levels.

U.S.P. 3,663,684, entitled "Carcinoembryonic Antigen and Diagnostic Method Using Radioactive Iodine", concerns purification and radioiodination of CEA for use in a RIA.

U.S.P. 3,697,638 describes that CEA is a mixture of antigens (components A and B in this case). U.S.P. 3,697,638 mentions methods for separating and radioiodinating each component and their use in specific RIA's.

U.S.P. 3,852,415, entitled "Compositions for Use in Radioimmunoassay, as Substitute for Blood Plasma Extract in Determination of Carcinoembryonic Antigen" relates to the use of a buffer containing EDTA and bovine serum albumin as a substitute for plasma as a diluent for CEA RIA's.

U.S.P. 3,867,363, entitled "Carcinoembryonic Antigens", is directed to the isolation of CEA components A and B, their labelling and use in a RIA.

U.S.P. 3,927,193, entitled "Localization of Tumors by Radiolabelled Antibodies", concerns the use of radiolabelled anti-CEA antibodies in whole body tumor imaging.

U.S.P. 3,956,258, entitled "Carcinoembryonic Antigens", relates to the isolation of CEA components A and B.

U.S.P. 4,086,217, entitled "Carcinoembryonic Antigens", is directed to the isolation of CEA components A and B.

U.S.P. 4,140,753, entitled "Diagnostic Method and Reagent", concerns the purification of a CEA isomer called CEA-S1 and its use in a RIA.

U.S.P. 4,145,336, entitled "Carcinoembryonic Antigen Isomer", relates to the antigen CEA-S1.

U.S.P. 4,180,499, entitled "Carcinoembryonic Antigens", describes a process for producing CEA component B.

U.S.P. 4,228,236, entitled "Process of Producing Carcinoembryonic Antigen", is directed to the use of the established cell lines LS-174T and LS-180 or clones or derivatives thereof for the production of CEA.

U.S.P. 4,272,504, entitled "Antibody Adsorbed Support Method for Carcinoembryonic Antigen Assay", concerns two concepts for the radioimmunoassay of CEA. First, U.S.P. 4,272,504 relates to a sample pretreatment in the form of heating to 65 to 85°C at pH 5 to precipitate and eliminate extraneous protein. Second, it describes the use of a solid phase antibody (either on beads or tubes) as a means to capture analyte and radiolabelled CEA tracer.

U.S.P. 4,299,815, entitled "Carcinoembryonic Antigen Determination", concerns diluting a CEA sample with water and pretreating by heating to a temperature below which precipitation of protein will occur. The pretreated sample is then immunoassayed using RIA, EIA, FIA or chemiluminescent immunoassay.

U.S.P. 4,349,528, entitled "Monoclonal Hybridoma Antibody Specific for High Molecular Weight Carcinoembryonic Antigen", is directed to a monoclonal antibody reacting with 180 kD CEA, but not with other molecular weight forms.

U.S.P. 4,467,031, entitled "Enzyme-Immunoassay for Carcinoembryonic Antigen", relates to a sandwich enzyme immunoassay for CEA in which the first of two anti-CEA monoclonal antibodies is attached to a solid phase and the second monoclonal is conjugated with peroxidase.

U.S.P. 4,489,167, entitled "Methods and Compositions for Cancer Detection", describes that CEA shares an antigenic determinant with alpha-acid glycoprotein (AG), which is a normal component of human serum. The method described therein concerns a solid-phase sandwich enzyme immunoassay using as one antibody an antibody recognizing AG and another antibody recognizing CEA, but not AG.

U.S.P. 4,578,349, entitled "Immunoassay for Carcinoembryonic Antigen (CEA)", is directed to the use of high salt containing buffers as diluents in CEA immunoassays.

EP 113072-A, entitled "Assaying Blood Sample for Carcinoembryonic Antigen - After Removal of Interfering Materials by Incubation with Silica Gel", relates to the removal from a serum of a plasma sample of interfering substances by pretreatment with silica gel. The precleared sample is then subjected to an immunoassay.

EP 102008-A, entitled "Cancer Diagnostics Carcinoembryonic Antigen - Produced from Perchloric Acid Extracts Without Electrophoresis", relates to a procedure for the preparation of CEA from perchloric acid extracts, without the use of an electrophoresis step.

EP 92223-A, entitled "Determination of Carcinoembryonic Antigen in Cytosol or Tissue - for Therapy Control and Early Recognition of Regression", concerns an immunoassay of CEA, not in serum or plasma, but in the cytosol fraction of the tumor tissue itself.

EP 83103759.6, entitled "Cytosole-CEA-Measurement as Predictive Test in Carcinoma, Particularly Mammacarcinoma", is similar to EP 92223-A.

EP 83303759, entitled "Monoclonal Antibodies Specific to Carcinoembryonic Antigen", relates to the production of "CEA specific" monoclonal antibodies and their use in immunoassays.

WO 84/02983, entitled "Specific CEA-Family Antigens, Antibodies Specific Thereto and Their Methods of Use", is directed to the use of monoclonal antibodies to CEA-meconium (MA)-, and NCA-specific epitopes in immunoassays designed to selectively measure each of these individual components in a sample.

All of the heretofore CEA assays utilize either monoclonal or polyclonal antibodies which are generated by immunizing animals with the intact antigen of choice. None of them address the idea of making sequence specific antibodies for the detection of a unique primary sequence of the various antigens. They do not cover the use of any primary amino acid sequence for the production of antibodies to synthetic peptides or fragments of the natural product. They do not include the concept of using primary amino acid sequences to distinguish the CEA family members. None of them covers the use of DNA or RNA clones for isolating the genes with which to determine the primary sequence.

Heretofore there was no teaching covering the use of DNA or RNA clones to generate cells which express members of the CEA gene family, nor the use of these cells in generating or screening specific monoclonal antibodies.

## DEFINITIONS

| Nucleic Acid Abbreviations | |
|---|---|
| A | adenine |
| G | guanine |
| C | cytosine |
| T | thymidine |
| U | uracil |

| Amino Acid Abbreviations: | |
|---|---|
| Asp | aspartic acid |
| Asn | asparagine |
| Thr | threonine |
| Ser | serine |
| Glu | glutamic acid |
| Gln | glutamine |
| Pro | proline |
| Gly | glycine |
| Ala | alanine |
| Cys | cysteine |
| Val | valine |
| Met | methionine |
| Ile | isoleucine |
| Leu | leucine |
| Tyr | tyrosine |
| Phe | phenylalanine |
| Trp | tryptophan |
| Lys | lysine |
| His | histidine |
| Arg | arginine |

Nucleotide - A monomeric unit of DNA or RNA containing a sugar moiety (pentose), a phosphate, and a nitrogenous heterocyclic base. The base is linked to the sugar moiety via the glycosidic carbon (1′ carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA Sequence - A linear array of nucleotides connected one to the other by phosphodiester bonds between the 3′ and 5′ carbons of adjacent pentoses.

Functional equivalents - It is well known in the art that in a DNA sequence some nucleotides can be replaced without having an influence on the sequence of the expression product. With respect to the peptide this term means that one or more amino acids which have no function in a particular use can be deleted or replaced by another one.

Codon - A DNA sequence of three nucleotides (a triplet) which encodes through mRNA an amino acid, a translation start signal or a translation termination signal. For example, the nucleotide triplets TTA, TTG, CTT, CTC, CTA and CTG encode the amino acid leucine ("Leu"), TAG, TAA and TGA are translation stop signals and ATG is a translation start signal.

Reading Frame - The grouping of codons during translation of mRNA into amino acid sequences. During translation, the proper reading frame must be maintained. For example, the sequence GCTGGTTGTAAG may be translated in three reading frames or phases, each of which affords a different amino acid sequence

GCT GGT TGT AAG - Ala-Gly-Cys-Lys
G CTG GTT GTA AG - Leu-Val-Val
GC TGG TTG TAA G - Trp-Leu-(STOP).

Polypeptide - A linear array of amino acids connected one to the other by peptide bonds between the alpha-amino and carboxy groups of adjacent amino acids.

Genome - The entire DNA of a cell or a virus. It includes inter alia the structural genes coding for the polypeptides of the cell or virus, as well as its operator, promoter and ribosome binding and interaction sequences, including sequences such as the Shine-Dalgarno sequences.

Structural Gene - A DNA sequence which encodes through its template or messenger RNA ("mRNA") a sequence of amino acids characteristic of a specific polypeptide.

Transcription - The process of producing mRNA from a structural gene.

Translation - The process of producing a polypeptide from mRNA.

Expression - The process undergone by a structural gene to produce a polypeptide. It is a combination of transcription and translation.

Plasmid - A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance ($Tet^R$) transforms a cell previously sensitive to tetracycline into one which is resistant to it. A cell transformed by a plasmid is called a "transformant".

Phage or Bacteriophage - Bacterial virus, many of which consist of DNA sequences encapsulated in a protein envelope or coat ("capsid protein").

Cloning Vehicle - A plasmid, phage DNA or other DNA sequence which is capable of replicating in a host cell, which is characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is often called a vector.

Cloning - The process of obtaining a population of organisms or DNA sequences derived from one such organism or sequence by asexual reproduction.

Recombinant DNA Molecule or Hybrid DNA - A molecule consisting of segments of DNA from different genomes which have been joined end-to-end outside of living cells and have the capacity to infect some host cell and be maintained therein.

cDNA Expression Vector - A procaroytic cloning vehicle which also contains sequences of nucleotides that facilitate the expression of cDNA sequences in eucaroytic cells. These nucleotides include sequences that function as a eucaroytic promoter, alternative splice sites and polyadenylation signals.

Transformation/Transfection - DNA or RNA is introduced into cells in such a way as to allow gene expression. "Infected" referred to herein concerns the introduction of RNA or DNA by a viral vector into the host.

CEA antigen family (CEA gene family) - A set of genes (gene family) and their products (antigen family) that share nucleotide sequences homologous to partial cDNA LV-7 (CEA-(a)) and as a result of these similarities also share a subset of their antigenic epitopes. Examples of the CEA antigen family include CEA (= CEA-(b)), transmembrane CEA (TM CEA) = CEA-(c) and normal crossreacting antigen (NCA) (= CEA-(d)).

## SUMMARY OF THE INVENTION

The present invention provides non-human mammalian, e.g., mouse, hamster and rat, cell lines which express CEA family antigens at the cell surface.

The present invention also relates to antibodies (polyclonal and monoclonal) to specific CEA family members generated by using the cell lines of the present invention.

Still further, the present invention also concerns a general method for testing the aforementioned antibodies, or other antibodies directed to CEA antigens produced by other methods, for their specificity to different members of the CEA gene family (antigen family) (e.g., CEA-(b), CEA-(c), CEA-(d) CEA-(e), CEA-(f) or CEA-(g). The testing could be conducted by an indirect immunofluorescence assay and fluorescence-activated cell sorter (FACS) analysis of the individual cell lines.

Primary transfectant cell lines for genomic genes are obtained by gene transfection of human DNA into suitable non-human mammalian cells, e.g., mouse cells. Secondary transfectants, characterized by the virtual absence of any human DNA sequences, other than the gene coding for the protein of interest, can then be obtained by gene transfection of the primary transfectant DNA into suitable non-human mammalian cells. Further selection of highly expressing secondary transfectants can lead to the isolation of substantially homogeneous high expression cloned cell lines that express as much as twenty fold or more CEA family antigens as secondary lines which have not been selected for amplification (M. E. Kamarck et al, Proc. Natl. Acad. Sci., U.S.A., 84, 5350-5354, August 1987).

In an antigen family such as CEA, where coding sequences are highly homologous, the same cDNA fragment can be used to isolate all members of the family. For example, CEA-(a), which is a partial cDNA, was used to isolate complete cDNA's for CEA family members CEA-(b), CEA-(c), CEA-(d), CEA-(e) and CEA-(f). The complete cDNA's (CEA-(b) (N. Beauchemin et al, Molecular and Cellular Biology, Vol. 7, No. 9, Sept. 1987, 3321-3230), CEA-(c), CEA-(d), CEA-(e) and CEA-(f) can then be cloned into cDNA expression vectors and transfected into non-human mammalian, for example, mouse cells. The cell lines that express CEA family antigens on the surface can be detected, selected and cloned using indirect immunofluorescence and FACS analysis.

## DETAILED DESCRIPTION OF THE INVENTION

In parent applications, applicants described the following CEA's:

| | |
|---|---|
| CEA-(a) | partial CEA |
| CEA-(b) | full coding CEA |
| CEA-(c) | TM-1 |
| CEA-(d) | NCA. |

In an application filed on even date herewith, applicants described the following CEA's:

| | |
|---|---|
| CEA-(e) | TM-2 |
| CEA-(f) | TM-3 |
| CEA-(g) | TM-4. |

The sequences for CEA-(a) to CEA-(g) are given hereinbelow:

(a)

| | | | | | | |
|---|---|---|---|---|---|---|
| 1 | GGGGTTTACA | CAACCACCAC | CCCATCAAAC | CCTTCATCAC | CAGCAACAAC | TCCAACCCCG |
| 61 | TGGAGGATGA | GGATGCTGTA | GCCTTAACCT | GTGAACCTGA | GATTCAGAAC | ACAACCTACC |
| 121 | TGTGGTGGGT | AAATAATCAG | AGCCTCCCGG | TCAGTCCCAG | GCTGCAGCTG | TCCAATGACA |
| 181 | ACAGGACCCT | CACTCTACTC | AGTGTCACAA | GGAATGATGT | AGGACCCTAT | GAGTGTGGAA |
| 241 | TCCAGAACGA | ATTAAGTGTT | GACCACAGCG | ACCCAGTCAT | CCTGAATGTC | CTCTATGGCC |
| 301 | CAGACGACCC | CACCATTTCC | CCCTCATACA | CCTATTACCG | TCCAGGGGTG | AACCTCAGCC |
| 361 | TCTCCTGCCA | TGCAGCCTCT | AACCCACCTG | CACAGTATTC | TTGGCTGATT | GATGGGAACA |
| 421 | TCCAGCAACA | CACACAAGAG | CTCTTTATCT | CCAACATCAC | TGAGAAGAAC | AGCGGACTCT |
| 481 | ATACCTGCCA | GGCCAATAAC | TCAGCCAGTG | GCCACAGCAG | GACTACAGTC | AAGACAATCA |
| 541 | CAGTCTCTGC | GGACGTGCCC | AAGCCCTCCA | TCTCCAGCAA | CAACTCCAAA | CCCGTGGAGG |
| 601 | ACAAGGATGC | TGTGGCCTTC | CACTGTGAAC | CTGAGGCTCA | GAACACAACC | TACCTGTGGT |
| 661 | GGGTAAATGG | TCAGAGCCTC | CCAGTCAGTC | CCAGGCTGCA | GCTGTCCAAT | GGCAACAGGA |
| 721 | CCCTCACTCT | ATTCAATGTC | ACAAGAAATG | ACGCAAGAGC | CTATGTATGT | GGAATCCAGA |
| 781 | ACTCAGTGAG | TGCAAACCGC | AGTGACCCAG | TCACCCTGGA | TGTCCTCTAT | GGGCCGGACA |
| 841 | CCCCCATCAT | TTCCCCCCCC | CC | | | |

(b)

```
            10          20          30          40          50
C ACC ATG GAG TCT CCC TCG GCC CCT CTC CAC AGA TGG TGC ATC CCC TGG CAG AGG CTC
      Met Glu Ser Pro Ser Ala Pro Leu His Arg Trp Cys Ile Pro Trp Gln Arg Leu
```

```
60        70        80        90        100       110
CTG CTC ACA GCC TCA CTT CTA ACC TTC TGG AAC CCG CCC ACC ACT GCC AAG CTC ACT
Leu Leu Thr Ala Ser Leu Leu Thr Phe Trp Asn Pro Pro Thr Thr Ala Lys Leu Thr
                                                                 1   2   3

120       130       140       150       160       170
ATT GAA TCC ACG CCG TTC AAT GTC GCA GAG GGG AAG GAG GTG CTT CTA CTT GTC CAC
Ile Glu Ser Thr Pro Phe Asn Val Ala Glu Gly Lys Glu Val Leu Leu Leu Val His
  4   5   6   7   8   9  10  11  12  13  14  15  16  17  18  19  20  21  22

180       190       200       210       220
AAT CTG CCC CAG CAT CTT TTT GGC TAC AGC TGG TAC AAA GGT GAA AGA GTG GAT GGC
Asn Leu Pro Gln His Leu Phe Gly Tyr Ser Trp Tyr Lys Gly Glu Arg Val Asp Gly
 23  24  25  26  27  28  29  30  31  32  33  34  35  36  37  38  39  40  41

230       240       250       260       270       280
AAC CGT CAA ATT ATA GGA TAT GTA ATA GGA ACT CAA CAA GCT ACC CCA GGG CCC GCA
Asn Arg Gln Ile Ile Gly Tyr Val Ile Gly Thr Gln Gln Ala Thr Pro Gly Pro Ala
 42  43  44  45  46  47  48  49  50  51  52  53  54  55  56  57  58  59  60

290       300       310       320       330       340
TAC AGT GGT CGA GAG ATA ATA TAC CCC AAT GCA TCC CTG CTG ATC CAG AAC ATC ATC
Tyr Ser Gly Arg Glu Ile Ile Tyr Pro Asn Ala Ser Leu Leu Ile Gln Asn Ile Ile
 61  62  63  64  65  66  67  68  69  70  71  72  73  74  75  76  77  78  79

350       360       370       380       390       400
CAG AAT GAC ACA GGA TTC TAC ACC CTA CAC GTC ATA AAG TCA GAT CTT GTG AAT GAA
Gln Asn Asp Thr Gly Phe Tyr Thr Leu His Val Ile Lys Ser Asp Leu Val Asn Glu
 80  81  82  83  84  85  86  87  88  89  90  91  92  93  94  95  96  97  98

410       420       430       440       450
GAA GCA ACT GGC CAG TTC CGG GTA TAC CCG GAG CTG CCC AAG CCC TCC ATC TCC AGC
Glu Ala Thr Gly Gln Phe Arg Val Tyr Pro Glu Leu Pro Lys Pro Ser Ile Ser Ser
 99 101 101 102 103 104 105 106 107 108 109 110 111 112 113 114 115 116 117

460       470       480       490       500       510
AAC AAC TCC AAA CCC GTG GAG GAC AAG GAT GCT GTG GCC TTC ACC TGT GAA CCT GAG
Asn Asn Ser Lys Pro Val Glu Asp Lys Asp Ala Val Ala Phe Thr Cys Glu Pro Glu
118 119 120 121 122 123 124 125 126 127 128 129 130 131 132 133 134 135 136
```

520 530 540 550 560 570

ACT CAG GAC GCA ACC TAC CTG TGG TGG GTA AAC AAT CAG AGC CTC CCG GTC AGT CCC

Thr Gln Asp Ala Thr Tyr Leu Trp Trp Val Asn Asn Gln Ser Leu Pro Val Ser Pro

137 138 139 140 141 142 143 144 145 146 147 148 149 150 151 152 153 154 155

580 590 600 610 620

AGG CTG CAG CTG TCC AAT GGC AAC AGG ACC CTC ACT CTA TTC AAT GTC ACA AGA AAT

Arg Leu Gln Leu Ser Asn Gly Asn Arg Thr Leu Thr Leu Phe Asn Val Thr Arg Asn

156 157 158 159 160 161 162 163 164 165 166 167 168 169 170 171 172 173 174

630 640 650 660 670 680

GAA CAA GCA AGC TAC AAA TGT GAA ACC CAG AAC CCA GTG AGT GCC AGG CGC AGT GAT

Glu Gln Ala Ser Tyr Lys Cys Glu Thr Gln Asn Pro Val Ser Ala Arg Arg Ser Asp

175 176 177 178 179 180 181 182 183 184 185 186 187 188 189 190 191 192 193

690 700 710 720 730 740

TCA GTC ATC CTG AAT GTC CTC TAT GGC CCG GAT GCC CCC ACC ATT TCC CCT CTA AAC

Ser Val Ile Leu Asn Val Leu Tyr Gly Pro Asp Ala Pro Thr Ile Ser Pro Leu Asn

194 195 196 197 198 199 200 201 202 203 204 205 206 207 208 209 210 211 212

750 760 770 780 790

ACA TCT TAC AGA TCA GGG GAA AAT CTG AAC CTC TCC TGC CAC GCA GCC TCT AAC CCA

Thr Ser Tyr Arg Ser Gly Glu Asn Leu Asn Leu Ser Cys His Ala Ala Ser Asn Pro

213 214 215 216 217 218 219 220 221 222 223 224 225 226 227 228 229 230 231

800 810 820 830 840 850

CCT GCA CAG TAC TCT TGG TTT GTC AAT GGG ACT TTC CAG CAA TCC ACC CAA GAG CTC

Pro Ala Gln Tyr Ser Trp Phe Val Asn Gly Thr Phe Gln Gln Ser Thr Gln Glu Leu

232 233 234 235 236 237 238 239 240 241 242 243 244 245 246 247 248 249 250

860 870 880 890 900 910

TTT ATC CCC AAC ATC ACT GTG AAT AAT AGT GGA TCC TAT ACG TGC CAA GCC CAT AAC

Phe Ile Pro Asn Ile Thr Val Asn Asn Ser Gly Ser Tyr Thr Cys Gln Ala His Asn

251 252 253 254 255 256 257 258 259 260 261 262 263 264 265 266 267 268 269

920 930 940 950 960 970

TCA GAC ACT GGC CTC AAT AGG ACC ACA GTC ACG ACG ATC ACA GTC TAT GCA GAG CCA

Ser Asp Thr Gly Leu Asn Arg Thr Thr Val Thr Thr Ile Thr Val Tyr Ala Glu Pro

270 271 272 273 274 275 276 277 278 279 280 281 282 283 284 285 286 287 288

980 990 1000 1010 1020

CCC AAA CCC TTC ATC ACC AGC AAC AAC TCC AAC CCC GTG GAG GAT GAG GAT GCT GTA
Pro Lys Pro Phe Ile Thr Ser Asn Asn Ser Asn Pro Val Glu Asp Glu Asp Ala Val
289 290 291 292 293 294 295 296 297 298 299 300 301 302 303 304 305 306 307

1030 1040 1050 1060 1070 1080

GCC TTA ACC TGT GAA CCT GAG ATT CAG AAC ACA ACC TAC CTG TGG TGG GTA AAT AAT
Ala Leu Thr Cys Glu Pro Glu Ile Gln Asn Thr Thr Tyr Leu Trp Trp Val Asn Asn
308 309 310 311 312 313 314 315 316 317 318 319 320 321 322 323 324 325 326

1090 1100 1110 1120 1130 1140

CAG AGC CTC CCG GTC AGT CCC AGG CTG CAG CTG TCC AAT GAC AAC AGG ACC CTC ACT
Gln Ser Leu Pro Val Ser Pro Arg Leu Gln Leu Ser Asn Asp Asn Arg Thr Leu Thr
327 328 329 330 331 332 333 334 335 336 337 338 339 340 341 342 343 344 345

1150 1160 1170 1180 1190

CTA CTC AGT GTC ACA AGG AAT GAT GTA GGA CCC TAT GAG TGT GGA ATC CAG AAC GAA
Leu Leu Ser Val Thr Arg Asn Asp Val Gly Pro Tyr Glu Cys Gly Ile Gln Asn Glu
346 347 348 349 350 351 352 353 354 355 356 357 358 359 360 361 362 363 364

1200 1210 1220 1230 1240 1250

TTA AGT GTT GAC CAC AGC GAC CCA GTC ATC CTG AAT GTC CTC TAT GGC CCA GAC GAC
Leu Ser Val Asp His Ser Asp Pro Val Ile Leu Asn Val Leu Tyr Gly Pro Asp Asp
365 366 367 368 369 370 371 372 373 374 375 376 377 378 379 380 381 382 383

1260 1270 1280 1290 1300 1310

CCC ACC ATT TCC CCC TCA TAC ACC TAT TAC CGT CCA GGG GTG AAC CTC AGC CTC TCC
Pro Thr Ile Ser Pro Ser Tyr Thr Tyr Tyr Arg Pro Gly Val Asn Leu Ser Leu Ser
384 385 386 387 388 389 390 391 392 393 394 395 396 397 398 399 400 401 402

1320 1330 1340 1350 1360

TGC CAT GCA GCC TCT AAC CCA CCT GCA CAG TAT TCT TGG CTG ATT GAT GGG AAC ATC
Cys His Ala Ala Ser Asn Pro Pro Ala Gln Tyr Ser Trp Leu Ile Asp Gly Asn Ile
403 404 405 406 407 408 409 410 411 412 413 414 415 416 417 418 419 420 421

1370 1380 1390 1400 1410 1420

CAG CAA CAC ACA CAA GAG CTC TTT ATC TCC AAC ATC ACT GAG AAG AAC AGC GGA CTC
Gln Gln His Thr Gln Glu Leu Phe Ile Ser Asn Ile Thr Glu Lys Asn Ser Gly Leu
422 423 424 425 426 427 428 429 430 431 432 433 434 435 436 437 438 439 440

```
 1430      1440      1450      1460      1470      1480
   *         *         *         *         *         *
TAT ACC TGC CAG GCC AAT AAC TCA GCC AGT GGC CAC AGC AGG ACT ACA GTC AAG ACA
Tyr Thr Cys Gln Ala Asn Asn Ser Ala Ser Gly His Ser Arg Thr Thr Val Lys Thr
441 442 443 444 445 446 447 448 449 450 451 452 453 454 455 456 457 458 459

    1490      1500      1510      1520      1530      1540
      *         *         *         *         *         *
ATC ACA GTC TCT GCG GAC GTG CCC AAG CCC TCC ATC TCC AGC AAC AAC TCC AAA CCC
Ile Thr Val Ser Ala Asp Val Pro Lys Pro Ser Ile Ser Ser Asn Asn Ser Lys Pro
460 461 462 463 464 465 466 467 468 469 470 471 472 473 474 475 476 477 478

       1550      1560      1570      1580      1590
         *         *         *         *         *
GTG GAG GAC AAG GAT GCT GTG GCC TTC ACC TGT GAA CCT GAG GCT CAG AAC ACA ACC
Val Glu Asp Lys Asp Ala Val Ala Phe Thr Cys Glu Pro Glu Ala Gln Asn Thr Thr
479 480 481 482 483 484 485 486 487 488 489 490 491 492 493 494 495 496 497

1600      1610      1620      1630      1640      1650
  *         *         *         *         *         *
TAC CTG TGG TGG GTA AAT GGT CAG AGC CTC CCA GTC AGT CCC AGG CTG CAG CTG TCC
Tyr Leu Trp Trp Val Asn Gly Gln Ser Leu Pro Val Ser Pro Arg Leu Gln Leu Ser
498 499 500 501 502 503 504 505 506 507 508 509 510 511 512 513 514 515 516

    1660      1670      1680      1690      1700      1710
      *         *         *         *         *         *
AAT GGC AAC AGG ACC CTC ACT CTA TTC AAT GTC ACA AGA AAT GAC GCA AGA GCC TAT
Asn Gly Asn Arg Thr Leu Thr Leu Phe Asn Val Thr Arg Asn Asp Ala Arg Ala Tyr
517 518 519 520 521 522 523 524 525 526 527 528 529 530 531 532 533 534 535

       1720      1730      1740      1750      1760
         *         *         *         *         *
GTA TGT GGA ATC CAG AAC TCA GTG AGT GCA AAC CGC AGT GAC CCA GTC ACC CTG GAT
Val Cys Gly Ile Gln Asn Ser Val Ser Ala Asn Arg Ser Asp Pro Val Thr Leu Asp
536 537 538 539 540 541 542 543 544 545 546 547 548 549 550 551 552 553 554

1770      1780      1790      1800      1810      1820
  *         *         *         *         *         *
GTC CTC TAT GGG CCG GAC ACC CCC ATC ATT TCC CCC CCA GAC TCG TCT TAC CTT TCG
Val Leu Tyr Gly Pro Asp Thr Pro Ile Ile Ser Pro Pro Asp Ser Ser Tyr Leu Ser
555 556 557 558 559 560 561 562 563 564 565 566 567 568 569 570 571 572 573

    1830      1840      1850      1860      1870      1880
      *         *         *         *         *         *
GGA GCG AAC CTC AAC CTC TCC TGC CAC TCG GCC TCT AAC CCA TCC CCG CAG TAT TCT
Gly Ala Asn Leu Asn Leu Ser Cys His Ser Ala Ser Asn Pro Ser Pro Gln Tyr Ser
574 575 576 577 578 579 580 581 582 583 584 585 586 587 588 589 590 591 592
```

```
         1890        1900        1910        1920        1930
           *           *           *           *           *
TGG CGT ATC AAT GGG ATA CCG CAG CAA CAC ACA CAA GTT CTC TTT ATC GCC AAA ATC
Trp Arg Ile Asn Gly Ile Pro Gln Gln His Thr Gln Val Leu Phe Ile Ala Lys Ile
593 594 595 596 597 598 599 600 601 602 603 604 605 606 607 608 609 610 611

1940        1950        1960        1970        1980        1990
   *           *           *           *           *           *
ACG CCA AAT AAT AAC GGG ACC TAT GCC TGT TTT GTC TCT AAC TTG GCT ACT GGC CGC
Thr Pro Asn Asn Asn Gly Thr Tyr Ala Cys Phe Val Ser Asn Leu Ala Thr Gly Arg
612 613 614 615 616 617 618 619 620 621 622 623 624 625 626 627 628 629 630

    2000        2010        2020        2030        2040        2050
       *           *           *           *           *           *
AAT AAT TCC ATA GTC AAG AGC ATC ACA GTC TCT GCA TCT GGA ACT TCT CCT GGT CTC
Asn Asn Ser Ile Val Lys Ser Ile Thr Val Ser Ala Ser Gly Thr Ser Pro Gly Leu
631 632 633 634 635 636 637 638 639 640 641 642 643 644 645 646 647 648 649

        2060        2070        2080        2090        2100        2110
           *           *           *           *           *           *
TCA GCT GGG GCC ACT GTC GGC ATC ATG ATT GGA GTG CTG GTT GGG GTT GCT CTG ATA
Ser Ala Gly Ala Thr Val Gly Ile Met Ile Gly Val Leu Val Gly Val Ala Leu Ile
650 651 652 653 654 655 656 657 658 659 660 661 662 663 664 665 666 667 668

            2120        2130        2140        2150        2160
               *           *           *           *           *
TAG CAG CCC TGG TGT AGT TTC TTC ATT TCA GGA AGA CTG ACA GTT GTT TTG CTT CTT

2170        2180        2190        2200        2210        2220
   *           *           *           *           *           *
CCT TAA AGC ATT TGC AAC AGC TAC AGT CTA AAA TTG CTT CTT TAC CAA GGA TAT TTA

    2230        2240        2250        2260        2270        2280
       *           *           *           *           *           *
CAG AAA ATA CTC TGA CCA GAG ATC GAG ACC ATC CTA GCC AAC ATC GTG AAA CCC CAT

        2290        2300        2310        2320        2330
           *           *           *           *           *
CTC TAC TAA AAA TAC AAA AAT GAG CTG GGC TTG GTG GCG CGC ACC TGT AGT CCC AGT

2340        2350        2360        2370        2380        2390
   *           *           *           *           *           *
TAC TCG GGA GGC TGA GGC AGG AGA ATC GCT TGA ACC CGG GAG GTG GAG ATT GCA GTG
```

```
    2400       2410       2420       2430       2440       2450
      *          *          *          *          *          *
AGC CCA GAT CGC ACC ACT GCA CTC CAG TCT GGC AAC AGA GCA AGA CTC CAT CTC AAA

        2460       2470       2480       2490       2500
          *          *          *          *          *
AAG AAA AGA AAA GAA GAC TCT GAC CTG TAC TCT TGA ATA CAA GTT TCT GAT ACC ACT

2510       2520       2530       2540       2550       2560
  *          *          *          *          *          *
GCA CTG TCT GAG AAT TTC CAA AAC TTT AAT GAA CTA ACT GAC AGC TTC ATG AAA CTG

   2570       2580       2590       2600       2610       2620
     *          *          *          *          *          *
TCC ACC AAG ATC AAG CAG AGA AAA TAA TTA ATT TCA TGG GGA CTA AAT GAA CTA ATG

        2630       2640       2650       2660       2670       2680
          *          *          *          *          *          *
AGG ATA ATA TTT TCA TAA TTT TTT ATT TGA AAT TTT GCT GAT TCT TTA AAT GTC TTG

            2690       2700       2710       2720       2730
              *          *          *          *          *
TTT CCC AGA TTT CAG GAA ACT TTT TTT CTT TTA AGC TAT CCA CTC TTA CAG CAA TTT

2740       2750       2760       2770       2780       2790
  *          *          *          *          *          *
GAT AAA ATA TAC TTT TGT GAA CAA AAA TTG AGA CAT TTA CAT TTT ATC CCT ATG TGG

     2800       2810       2820       2830
       *          *          *          *
TCG CTC CAG ACT TGG GAA ACT ATT CAT GAA TAT TTA TAT TGT ATG
```

CEA-(c):

```
        10                  30                  50
         .        .        .        .        .        .
CAGCCGTGCTCGAAGCGTTCCTGGAGCCCAAGCTCTCCTCCACAGGTGAAGACAGGGCCA

        70                  90                 110
         .        .        .        .        .        .
GCAGGAGACACCATGGGGCACCTCTCAGCCCCACTTCACAGAGTGCGTGTACCCTGGCAG
             MetGlyHisLeuSerAlaProLeuHisArgValArgValProTrpGln

       130                 150                 170
         .        .        .        .        .        .
GGGCTTCTGCTCACAGCCTCACTTCTAACCTTCTGGAACCCGCCCACCACTGCCCAGCTC
GlyLeuLeuLeuThrAlaSerLeuLeuThrPheTrpAsnProProThrThrAlaGlnLeu

       190                 210                 230
         .        .        .        .        .        .
ACTACTGAATCCATGCCATTCAATGTTGCAGAGGGGAAGGAGGTTCTTCTCCTTGTCCAC
ThrThrGluSerMetProPheAsnValAlaGluGlyLysGluValLeuLeuLeuValHis

       250                 270                 290
         .        .        .        .        .        .
AATCTGCCCCAGCAACTTTTTGGCTACAGCTGGTACAAAGGGGAAAGAGTGGATGGCAAC
AsnLeuProGlnGlnLeuPheGlyTyrSerTrpTyrLysGlyGluArgValAspGlyAsn

       310                 330                 350
         .        .        .        .        .        .
CGTCAAATTGTAGGATATGCAATAGGAACTCAACAAGCTACCCCAGGGCCCGCAAACAGC
ArgGlnIleValGlyTyrAlaIleGlyThrGlnGlnAlaThrProGlyProAlaAsnSer

       370                 390                 410
         .        .        .        .        .        .
GGTCGAGAGACAATATACCCCAATGCATCCCTGCTGATCCAGAACGTCACCCAGAATGAC
GlyArgGluThrIleTyrProAsnAlaSerLeuLeuIleGlnAsnValThrGlnAsnAsp

       430                 450                 470
         .        .        .        .        .        .
ACAGGATTCTACACCCTACAAGTCATAAAGTCAGATCTTGTGAATGAAGAAGCAACTGGA
ThrGlyPheTyrThrLeuGlnValIleLysSerAspLeuValAsnGluGluAlaThrGly

       490                 510                 530
         .        .        .        .        .        .
CAGTTCCATGTATACCCGGAGCTGCCCAAGCCCTCCATCTCCAGCAACAACTCCAACCCT
GlnPheHisValTyrProGluLeuProLysProSerIleSerSerAsnAsnSerAsnPro

       550                 570                 590
         .        .        .        .        .        .
GTGGAGGACAAGGATGCTGTGGCCTTCACCTGTGAACCTGAGACTCAGGACACAACCTAC
ValGluAspLysAspAlaValAlaPheThrCysGluProGluThrGlnAspThrThrTyr

       610                 630                 650
         .        .        .        .        .
```

```
CTGTGGTGGATAAACAATCAGAGCCTCCCGGTCAGTCCCAGGCTGCAGCTGTCCAATGGC
LeuTrpTrpIleAsnAsnGlnSerLeuProValSerProArgLeuGlnLeuSerAsnGly

         670                 690                 710
          .         .         .         .         .         .
AACAGGACCCTCACTCTACTCAGTGTCACAAGGAATGACACAGGACCCTATGAGTGTGAA
AsnArgThrLeuThrLeuLeuSerValThrArgAsnAspThrGlyProTyrGluCysGlu

         730                 750                 770
          .         .         .         .         .         .
ATACAGAACCCAGTGAGTGCGAACCGCAGTGACCCAGTCACCTTGAATGTCACCTATGGC
IleGlnAsnProValSerAlaAsnArgSerAspProValThrLeuAsnValThrTyrGly

         790                 810                 830
          .         .         .         .         .         .
CCGGACACCCCCACCATTTCCCCTTCAGACACCTATTACCGTCCAGGGGCAAACCTCAGC
ProAspThrProThrIleSerProSerAspThrTyrTyrArgProGlyAlaAsnLeuSer

         850                 870                 890
          .         .         .         .         .         .
CTCTCCTGCTATGCAGCCTCTAACCCACCTGCACAGTACTCCTGGCTTATCAATGGAACA
LeuSerCysTyrAlaAlaSerAsnProProAlaGlnTyrSerTrpLeuIleAsnGlyThr

         910                 930                 950
          .         .         .         .         .         .
TTCCAGCAAAGCACACAAGAGCTCTTTATCCCTAACATCACTGTGAATAATAGTGGATCC
PheGlnGlnSerThrGlnGluLeuPheIleProAsnIleThrValAsnAsnSerGlySer

         970                 990                1010
          .         .         .         .         .         .
TATACCTGCCACGCCAATAACTCAGTCACTGGCTGCAACAGGACCACAGTCAAGACGATC
TyrThrCysHisAlaAsnAsnSerValThrGlyCysAsnArgThrThrValLysThrIle

        1030                1050                1070
          .         .         .         .         .         .
ATAGTCACTGAGCTAAGTCCAGTAGTAGCAAAGCCCCAAATCAAAGCCAGCAAGACCACA
IleValThrGluLeuSerProValValAlaLysProGlnIleLysAlaSerLysThrThr

        1090                1110                1130
          .         .         .         .         .         .
GTCACAGGAGATAAGGACTCTGTGAACCTGACCTGCTCCACAAATGACACTGGAATCTCC
ValThrGlyAspLysAspSerValAsnLeuThrCysSerThrAsnAspThrGlyIleSer

        1150                1170                1190
          .         .         .         .         .         .
```

```
ATCCGTTGGTTCTTCAAAAACCAGAGTCTCCCGTCCTCGGAGAGGATGAAGCTGTCCCAG
IleArgTrpPhePheLysAsnGlnSerLeuProSerSerGluArgMetLysLeuSerGln

        1210                1230                1250
           .         .         .         .         .         .
GGCAACACCACCCTCAGCATAAACCCTGTCAAGAGGGAGGATGCTGGGACGTATTGGTGT
GlyAsnThrThrLeuSerIleAsnProValLysArgGluAspAlaGlyThrTyrTrpCys

        1270                1290                1310
           .         .         .         .         .         .
GAGGTCTTCAACCCAATCAGTAAGAACCAAAGCGACCCCATCATGCTGAACGTAAACTAT
GluValPheAsnProIleSerLysAsnGlnSerAspProIleMetLeuAsnValAsnTyr

        1330                1350                1370
           .         .         .         .         .         .
AATGCTCTACCACAAGAAAATGGCCTCTCACCTGGGGCCATTGCTGGCATTGTGATTGGA
AsnAlaLeuProGlnGluAsnGlyLeuSerProGlyAlaIleAlaGlyIleValIleGly

        1390                1410                1430
           .         .         .         .         .         .
GTAGTGGCCCTGGTTGCTCTGATAGCAGTAGCCCTGGCATGTTTTCTGCATTTCGGGAAG
ValValAlaLeuValAlaLeuIleAlaValAlaLeuAlaCysPheLeuHisPheGlyLys

        1450                1470                1490
           .         .         .         .         .         .
ACCGGCAGGGCAAGCGACCAGCGTGATCTCACAGAGCACAAACCCTCAGTCTCCAACCAC
ThrGlyArgAlaSerAspGlnArgAspLeuThrGluHisLysProSerValSerAsnHis

        1510                1530                1550
           .         .         .         .         .         .
ACTCAGGACCACTCCAATGACCCACCTAACAAGATGAATGAAGTTACTTATTCTACCCTG
ThrGlnAspHisSerAsnAspProProAsnLysMetAsnGluValThrTyrSerThrLeu

        1570                1590                1610
           .         .         .         .         .         .
AACTTTGAAGCCCAGCAACCCACACAACCAACTTCAGCCTCCCCATCCCTAACAGCCACA
AsnPheGluAlaGlnGlnProThrGlnProThrSerAlaSerProSerLeuThrAlaThr

        1630                1650                1670
           .         .         .         .         .         .
GAAATAATTTATTCAGAAGTAAAAAAGCAGTAATGAAACCTGTCCTGCTCACTGCAGTGC
GluIleIleTyrSerGluValLysLysGln

        1690                1710                1730
           .         .         .         .         .         .
TGATGTATTTCAAGTCTCTCACCCTCATCACTAGGAGATTCCTTTCCCCTGTAGGGTAGA

        1750                1770                1790
           .         .         .         .         .         .
GGGGTGGGGACAGAAACAACTTTCTCCTACTCTTCCTTCCTAATAGGCATCTCCAGGCTG

        1810                1830                1850
           .         .         .         .         .         .
CCTGGTCACTGCCCCTCTCTCAGTGTCAATAGATGAAAGTACATTGGGAGTCTGTAGGAA
```

1870 1890 1910

ACCCAACCTTCTTGTCATTGAAATTTGGCAAAGCTGACTTTGGGAAAGAGGGACCAGAAC

1930 1950 1970

TTCCCCTCCCTTCCCCTTTTCCCAACCTGGACTTGTTTTAAACTTGCCTGTTCAGAGCAC

1990 2010 2030

TCATTCCTTCCCACCCCCAGTCCTGTCCTATCACTCTAATTCGGATTTGCCATAGCCTTG

2050 2070 2090

AGGTTATGTCCTTTTCCATTAAGTACATGTGCCAGGAAACAGCGAGAGAGAGAAAGTAAA

2110 2130 2150

CGGCAGTAATGCTTCTCCTATTTCTCCAAAGCCTTGTGTGAACTAGCAAAGAGAAGAAAA

2170 2190 2210

TCAAATATATAACCAATAGTGAAATGCCACAGGTTTGTCCACTGTCAGGGTTGTCTACCT

2230 2250 2270

GTAGGATCAGGGTCTAAGCACCTTGGTGCTTAGCTAGAATACCACCTAATCCTTCTGGCA

2290 2310 2330

AGCCTGTCTTCAGAGAACCCACTAGAAGCAACTAGGAAAAATCACTTGCCAAAATCCAAG

2350 2370 2390

GCAATTCCTGATGGAAAATGCAAAAGCACATATATGTTTTAATATCTTTATGGGCTCTGT

2410 2430 2450

TCAAGGCAGTGCTGAGAGGGAGGGGTTATAGCTTCAGGAGGGAACCAGCTTCTGATAAAC

2470 2490 2510

ACAATCTGCTAGGAACTTGGGAAAGGAATCAGAGAGCTGCCCTTCAGCGATTATTTAAAT

2530 2550 2570

TGTTAAAGAATACACAATTTGGGGTATTGGGATTTTTCTCCTTTTCTCTGAGACATTCCA

2590 2610 2630

CCATTTTAATTTTTGTAACTGCTTATTTATGTGAAAAGGGTTATTTTTACTTAGCTTAGC

2650 2670 2690

TATGTCAGCCAATCCGATTGCCTTAGGTGAAAGAAACCACCGAAATCCCTCAGGTCCCTT

2710 2730 2750

GGTCAGGAGCCTCTCAAGATTTTTTTGTCAGAGGCTCCAAATAGAAAATAAGAAAAGGT

2770 2790 2810

TTTCTTCATTCATGGCTAGAGCTAGATTTAACTCAGTTTCTAGGCACCTCAGACCAATCA

2830 2850 2870

TCAACTACCATTCTATTCCATGTTTGCACCTGTGCATTTTCTGTTTGCCCCCATTCACTT

2890 2910 2930

TGTCAGGAAACCTTGGCCTCTGCTAAGGTGTATTTGGTCCTTGAGAAGTGGGAGCACCCT

2950 2970 2990

ACAGGGACACTATCACTCATGCTGGTGGCATTGTTTACAGCTAGAAAGCTGCACTGGTGC

3010 3030 3050

TAATGCCCCTTGGGAAATGGGGCTGTGAGGAGGAGGATTATAACTTAGGCCTAGCCTCTT

3070 3090 3110

TTAACAGCCTCTGAAATTTATCTTTTCTTCTATGGGGTCTATAAATGTATCTTATAATAA

3130 3150 3170

AAAGGAAGGACAGGAGGAAGACAGGCAAATGTACTTCTCACCCAGTCTTCTACACAGATG

3190 3210 3230

GAATCTCTTTGGGGCTAAGAGAAAGGTTTTATTCTATATTGCTTACCTGATCTCATGTTA

3250 3270 3290

GGCCTAAGAGGCTTTCTCCAGGAGGATTAGCTTGGAGTTCTCTATACTCAGGTACCTCTT

3310 3330 3350

TCAGGGTTTTCTAACCCTGACACGGACTGTGCATACTTTCCCTCATCCATGCTGTGCTGT

3370 3390 3410

GTTATTTAATTTTTCCTGGCTAAGATCATGTCTGAATTATGTATGAAAATTATTCTATGT

3430 3450

TTTTATAATAAAAATAATATATCAGACATCGAAAAAAAAAA

(d)

```
  10   20   30   40   50
CC GGG GGA CAC GCA GGG CCA ACA GTC ACA GCA GCC CTG ACC AGA GCA TTC CTG GAG CTC

60   70   80   90   100   110
AAG CTC TCT ACA AAG AGG TGG ACA GAG AAG ACA GCA GAG ACC ATG GGA CCC CCC TCA
                                                        Met Gly Pro Pro Ser

120   130   140   150   160   170
GCC CCT CCC TGC AGA TTG CAT GTC CCC TGG AAG GAG GTC CTG CTC ACA GCC TCA CTT
Ala Pro Pro Cys Arg Leu His Val Pro Trp Lys Glu Val Leu Leu Thr Ala Ser Leu

180   190   200   210   220   230
CTA ACC TTC TGG AAC CCA CCC ACC ACT GCC AAG CTC ACT ATT GAA TCC ACG CCA TTC
Leu Thr Phe Trp Asn Pro Pro Thr Thr Ala Lys Leu Thr Ile Glu Ser Thr Pro Phe
                                            1   2   3   4   5   6   7   8   9

240   250   260   270   280
AAT GTC GCA GAG GGG AAG GAG GTT CTT CTA CTC GCC CAC AAC CTG CCC CAG AAT CGT
Asn Val Ala Glu Gly Lys Glu Val Leu Leu Leu Ala His Asn Leu Pro Gln Asn Arg
10  11  12  13  14  15  16  17  18  19  20  21  22  23  24  25  26  27  28

290   300   310   320   330   340
ATT GGT TAC AGC TGG TAC AAA GGC GAA AGA GTG GAT GGC AAC AGT CTA ATT GTA GGA
Ile Gly Tyr Ser Trp Tyr Lys Gly Glu Arg Val Asp Gly Asn Ser Leu Ile Val Gly
29  30  31  32  33  34  35  36  37  38  39  40  41  42  43  44  45  46  47

350   360   370   380   390   400
TAT GTA ATA GGA ACT CAA CAA GCT ACC CCA GGG CCC GCA TAC AGT GGT CGA GAG ACA
Tyr Val Ile Gly Thr Gln Gln Ala Thr Pro Gly Pro Ala Tyr Ser Gly Arg Glu Thr
48  49  50  51  52  53  54  55  56  57  58  59  60  61  62  63  64  65  66

410   420   430   440   450
ATA TAC CCC AAT GCA TCC CTG CTG ATC CAG AAC GTC ACC CAG AAT GAC ACA GGA TTC
Ile Tyr Pro Asn Ala Ser Leu Leu Ile Gln Asn Val Thr Gln Asn Asp Thr Gly Phe
67  68  69  70  71  72  73  74  75  76  77  78  79  80  81  82  83  84  85

460   470   480   490   500   510
TAC ACC CTA CAA GTC ATA AAG TCA GAT CTT GTG AAT GAA GAA GCA ACC GGA CAG TTC
Tyr Thr Leu Gln Val Ile Lys Ser Asp Leu Val Asn Glu Glu Ala Thr Gly Gln Phe
86  87  88  89  90  91  92  93  94  95  96  97  98  99  100 101 102 103 104

520   530   540   550   560   570
CAT GTA TAC CCG GAG CTG CCC AAG CCC TCC ATC TCC AGC AAC AAC TCC AAC CCC GTG
His Val Tyr Pro Glu Leu Pro Lys Pro Ser Ile Ser Ser Asn Asn Ser Asn Pro Val
105 106 107 108 109 110 111 112 113 114 115 116 117 118 119 120 121 122 123
```

580 590 600 610 620

GAG GAC AAG GAT GCT GTG GCC TTC ACC TGT GAA CCT GAG GTT CAG AAC ACA ACC TAC
Glu Asp Lys Asp Ala Val Ala Phe Thr Cys Glu Pro Glu Val Gln Asn Thr Thr Tyr
124 125 126 127 128 129 130 131 132 133 134 135 136 137 138 139 140 141 142

630 640 650 660 670 680

CTG TGG TGG GTA AAT GGT CAG AGC CTC CCG GTC AGT CCC AGG CTG CAG CTG TCC AAT
Leu Trp Trp Val Asn Gly Gln Ser Leu Pro Val Ser Pro Arg Leu Gln Leu Ser Asn
143 144 145 146 147 148 149 150 151 152 153 154 155 156 157 158 159 160 161

690 700 710 720 730 740

GGC AAC AGG ACC CTC ACT CTA CTC AGC GTC AAA AGG AAC GAT GCA GGA TCG TAT GAA
Gly Asn Arg Thr Leu Thr Leu Leu Ser Val Lys Arg Asn Asp Ala Gly Ser Tyr Glu
162 163 164 165 166 167 168 169 170 171 172 173 174 175 176 177 178 179 180

750 760 770 780 790 800

TGT GAA ATA CAG AAC CCA GCG AGT GCC AAC CGC AGT GAC CCA GTC ACC CTG AAT GTC
Cys Glu Ile Gln Asn Pro Ala Ser Ala Asn Arg Ser Asp Pro Val Thr Leu Asn Val
181 182 183 184 185 186 187 188 189 190 191 192 193 194 195 196 197 198 199

810 820 830 840 850

CTC TAT GGC CCA GAT GGC CCC ACC ATT TCC CCC TCA AAG GCC AAT TAC CGT CCA GGG
Leu Tyr Gly Pro Asp Gly Pro Thr Ile Ser Pro Ser Lys Ala Asn Tyr Arg Pro Gly
200 201 202 203 204 205 206 207 208 209 210 211 212 213 214 215 216 217 218

860 870 880 890 900 910

GAA AAT CTG AAC CTC TCC TGC CAC GCA GCC TCT AAC CCA CCT GCA CAG TAC TCT TGG
Glu Asn Leu Asn Leu Ser Cys His Ala Ala Ser Asn Pro Pro Ala Gln Tyr Ser Trp
219 220 221 222 223 224 225 226 227 228 229 230 231 232 233 234 235 236 237

920 930 940 950 960 970

TTT ATC AAT GGG ACG TTC CAG CAA TCC ACA CAA GAG CTC TTT ATC CCC AAC ATC ACT
Phe Ile Asn Gly Thr Phe Gln Gln Ser Thr Gln Glu Leu Phe Ile Pro Asn Ile Thr
238 239 240 241 242 243 244 245 246 247 248 249 250 251 252 253 254 255 256

980 990 1000 1010 1020

GTG AAT AAT AGC GGA TCC TAT ATG TGC CAA GCC CAT AAC TCA GCC ACT GGC CTC AAT
Val Asn Asn Ser Gly Ser Tyr Met Cys Gln Ala His Asn Ser Ala Thr Gly Leu Asn
257 258 259 260 261 262 263 264 265 266 267 268 269 270 271 272 273 274 275

1030 1040 1050 1060 1070 1080

AGG ACC ACA GTC ACG ATG ATC ACA GTC TCT GGA AGT GCT CCT GTC CTC TCA GCT GTG
Arg Thr Thr Val Thr Met Ile Thr Val Ser Gly Ser Ala Pro Val Leu Ser Ala Val
276 277 278 279 280 281 282 283 284 285 286 287 288 289 290 291 292 293 294

1090 1100 1110 1120 1130 1140

GCC ACC GTC GGC ATC ACG ATT GGA GTG CTG GCC AGG GTG GCT CTG ATA TAG CAG CCC
Ala Thr Val Gly Ile Thr Ile Gly Val Leu Ala Arg Val Ala Leu Ile ---
295 296 297 298 299 300 301 302 303 304 305 306 307 308 309 310

1150 1160 1170 1180 1190

TGG TGT ATT TTC GAT ATT TCA GGA AGA CTG GCA GAT TGG ACC AGA CCC TGA ATT CTT

1200 1210 1220 1230 1240 1250

CTA GCT CCT CCA ATC CCA TTT TAT CCC ATG GAA CCA CTA AAA ACA AGG TCT GCT CTG

1260 1270 1280 1290 1300 1310

CTC CTG AAG CCC TAT ATG CTG GAG ATG GAC AAC TCA ATG AAA ATT TAA AGG AAA AAC

1320 1330 1340 1350 1360 1370

CCT CAG GCC TGA GGT GTG TGC CAC TCA GAG ACT TCA CCT AAC TAG AGA CAG GCA AAC

1380 1390 1400 1410 1420

TGC AAA CCA nnC CTC TTT CGC TTG GCA GGA TGA TGG TGT CAT TAG TAT TTC ACA AGA

1430 1440 1450 1460 1470 1480

AGT AGC TTC AGA GGG TAA CTT AAC AGA GTA TCA GAT CTA TCT TGT CAA TCC CAA CGT

1490 1500 1510 1520 1530 1540

TTT ACA TAA AAT AAG CGA TCC TTT AGT GCA CCC AGT GAC TGA CAT TAG CAG CAT CTT

1550 1560 1570 1580 1590

TAA CAC AGC CGT GTG TTC AAG TGT ACA GTG GTC CTT TTC AGA GTT GGn nnT ACT CCA

1600 1610 1620 1630 1640 1650

ACT GAA ATG TTA AGG AAG AAG ATA GAT CCA ATT AAA AAA AAT TAA nAC CAA TTT AAA

1660 1670 1680 1690 1700 1710

AAA AAA AAA GAA CAC AGG AGA TTC CAG TCT ACT TGA GTT AGC ATA ATA CAG AAG TCC

1720 1730 1740 1750 1760

CCT CTA CTT TAA CTT TTA CAA AAA AGT AAC CTG AAC TAA TCT GAT GTT AAC CAA TGT

```
1770         1780         1790         1800         1810         1820
 |            |            |            |            |            |
ATT TAT TTG TCT GGT TCT GTT TCC TTG TTC CAA TTT GAC AAA ACC CAC TGT TCT TGT

    1830         1840         1850         1860         1870         1880
     |            |            |            |            |            |
ATT GTA TTG CCC AGG GGG AGC TAT CAC TGT ACT TGT AGA GTG GTG CTG CTT TAA GTT

        1890         1900         1910         1920         1930         1940
         |            |            |            |            |            |
CAT AAA TCA CAA ATA AAA GCC AAT TAG CTC TAT AAC TAA AAA AAA AAA AAA AAA AAA

           1950         1960
            |            |
AAA AAA AAA AAA AAA AAA AAA AAA
```

, functional equivalents thereof and fragments thereof.

CEA-(e):

```
         10                   30                   50
          .         .         .         .         .         .
CAGCCGTGCTCGAAGCGTTCCTGGAGCCCAAGCTCTCCTCCACAGGTGAAGACAGGGCCA

         70                   90                  110
          .         .         .         .         .         .
GCAGGAGACACCATGGGGCACCTCTCAGCCCCACTTCACAGAGTGCGTGTACCCTGGCAG
            MetGlyHisLeuSerAlaProLeuHisArgValArgValProTrpGln

        130                  150                  170
          .         .         .         .         .         .
GGGCTTCTGCTCACAGCCTCACTTCTAACCTTCTGGAACCCGCCCACCACTGCCCAGCTC
GlyLeuLeuLeuThrAlaSerLeuLeuThrPheTrpAsnProProThrThrAlaGlnLeu

        190                  210                  230
          .         .         .         .         .         .
ACTACTGAATCCATGCCATTCAATGTTGCAGAGGGGAAGGAGGTTCTTCTCCTTGTCCAC
ThrThrGluSerMetProPheAsnValAlaGluGlyLysGluValLeuLeuLeuValHis

        250                  270                  290
          .         .         .         .         .         .
AATCTGCCCCAGCAACTTTTTGGCTACAGCTGGTACAAAGGGGAAAGAGTGGATGGCAAC
AsnLeuProGlnGlnLeuPheGlyTyrSerTrpTyrLysGlyGluArgValAspGlyAsn

        310                  330                  350
          .         .         .         .         .         .
CGTCAAATTGTAGGATATGCAATAGGAACTCAACAAGCTACCCCAGGGCCCGCAAACAGC
ArgGlnIleValGlyTyrAlaIleGlyThrGlnGlnAlaThrProGlyProAlaAsnSer

        370                  390                  410
          .         .         .         .         .         .
GGTCGAGAGACAATATACCCCAATGCATCCCTGCTGATCCAGAACGTCACCCAGAATGAC
GlyArgGluThrIleTyrProAsnAlaSerLeuLeuIleGlnAsnValThrGlnAsnAsp

        430                  450                  470
          .         .         .         .         .         .
ACAGGATTCTACACCCTACAAGTCATAAAGTCAGATCTTGTGAATGAAGAAGCAACTGGA
ThrGlyPheTyrThrLeuGlnValIleLysSerAspLeuValAsnGluGluAlaThrGly
```

```
     490                 510                 530
      .         .         .         .         .         .
CAGTTCCATGTATACCCGGAGCTGCCCAAGCCCTCCATCTCCAGCAACAACTCCAACCCT
GlnPheHisValTyrProGluLeuProLysProSerIleSerSerAsnAsnSerAsnPro

     550                 570                 590
      .         .         .         .         .         .
GTGGAGGACAAGGATGCTGTGGCCTTCACCTGTGAACCTGAGACTCAGGACACAACCTAC
ValGluAspLysAspAlaValAlaPheThrCysGluProGluThrGlnAspThrThrTyr

     610                 630                 650
      .         .         .         .         .         .
CTGTGGTGGATAAACAATCAGAGCCTCCCGGTCAGTCCCAGGCTGCAGCTGTCCAATGGC
LeuTrpTrpIleAsnAsnGlnSerLeuProValSerProArgLeuGlnLeuSerAsnGly

     670                 690                 710
      .         .         .         .         .         .
AACAGGACCCTCACTCTACTCAGTGTCACAAGGAATGACACAGGACCCTATGAGTGTGAA
AsnArgThrLeuThrLeuLeuSerValThrArgAsnAspThrGlyProTyrGluCysGlu

     730                 750                 770
      .         .         .         .         .         .
ATACAGAACCCAGTGAGTGCGAACCGCAGTGACCCAGTCACCTTGAATGTCACCTATGGC
IleGlnAsnProValSerAlaAsnArgSerAspProValThrLeuAsnValThrTyrGly

     790                 810                 830
      .         .         .         .         .         .
CCGGACACCCCCACCATTTCCCCTTCAGACACCTATTACCGTCCAGGGGCAAACCTCAGC
ProAspThrProThrIleSerProSerAspThrTyrTyrArgProGlyAlaAsnLeuSer

     850                 870                 890
      .         .         .         .         .         .
CTCTCCTGCTATGCAGCCTCTAACCCACCTGCACAGTACTCCTGGCTTATCAATGGAACA
LeuSerCysTyrAlaAlaSerAsnProProAlaGlnTyrSerTrpLeuIleAsnGlyThr

     910                 930                 950
      .         .         .         .         .         .
TTCCAGCAAAGCACACAAGAGCTCTTTATCCCTAACATCACTGTGAATAATAGTGGATCC
PheGlnGlnSerThrGlnGluLeuPheIleProAsnIleThrValAsnAsnSerGlySer

     970                 990                 1010
      .         .         .         .         .         .
TATACCTGCCACGCCAATAACTCAGTCACTGGCTGCAACAGGACCACAGTCAAGACGATC
TyrThrCysHisAlaAsnAsnSerValThrGlyCysAsnArgThrThrValLysThrIle
```

1030 1050 1070

ATAGTCACTGATAATGCTCTACCACAAGAAAATGGCCTCTCACCTGGGGCCATTGCTGGC
IleValThrAspAsnAlaLeuProGlnGluAsnGlyLeuSerProGlyAlaIleAlaGly

1090 1110 1130

ATTGTGATTGGAGTAGTGGCCCTGGTTGCTCTGATAGCAGTAGCCCTGGCATGTTTTCTG
IleValIleGlyValValAlaLeuValAlaLeuIleAlaValAlaLeuAlaCysPheLeu

1150 1170 1190

CATTTCGGGAAGACCGGCAGGGCAAGCGACCAGCGTGATCTCACAGAGCACAAACCCTCA
HisPheGlyLysThrGlyArgAlaSerAspGlnArgAspLeuThrGluHisLysProSer

1210 1230 1250

GTCTCCAACCACACTCAGGACCACTCCAATGACCCACCTAACAAGATGAATGAAGTTACT
ValSerAsnHisThrGlnAspHisSerAsnAspProProAsnLysMetAsnGluValThr

1270 1290 1310

TATTCTACCCTGAACTTTGAAGCCCAGCAACCCACACAACCAACTTCAGCCTCCCCATCC
TyrSerThrLeuAsnPheGluAlaGlnGlnProThrGlnProThrSerAlaSerProSer

1330 1350 1370

CTAACAGCCACAGAAATAATTTATTCAGAAGTAAAAAAGCAGTAATGAAACCTGTCCTGC
LeuThrAlaThrGluIleIleTyrSerGluValLysLysGln

1390 1410 1430

TCACTGCAGTGCTGATGTATTTCAAGTCTCTCACCCTCATCACTAGGAGATTCCTTTCCC

1450 1470 1490

CTGTAGGGTAGAGGGGTGGGGACAGAAACAACTTTCTCCTACTCTTCCTTCCTAATAGGC

1510 1530 1550

ATCTCCAGGCTGCCTGGTCACTGCCCCTCTCTCAGTGTCAATAGATGAAAGTACATTGGG

1570 1590 1610

AGTCTGTAGGAAACCCAACCTTCTTGTCATTGAAATTTGGCAAAGCTGACTTTGGGAAAG

```
        1630              1650              1670
AGGGACCAGAACTTCCCCTCCCTTCCCCTTTTCCCAACCTGGACTTGTTTTAAACTTGCC

        1690              1710              1730
TGTTCAGAGCACTCATTCCTTCCCACCCCCAGTCCTGTCCTATCACTCTAATTCGGATTT

        1750              1770              1790
GCCATAGCCTTGAGGTTATGTCCTTTTCCATTAAGTACATGTGCCAGGAAACAGCGAGAG

        1810              1830              1850
AGAGAAAGTAAACGGCAGTAATGCTTCTCCTATTTCTCCAAAGCCTTGTGTGAACTAGCA

        1870              1890              1910
AAGAGAAGAAAATCAAATATATAACCAATAGTGAAATGCCACAGGTTTGTCCACTGTCAG

        1930              1950              1970
GGTTGTCTACCTGTAGGATCAGGGTCTAAGCACCTTGGTGCTTAGCTAGAATACCACCTA

        1990              2010              2030
ATCCTTCTGGCAAGCCTGTCTTCAGAGAACCCACTAGAAGCAACTAGGAAAAATCACTTG

        2050              2070              2090
CCAAAATCCAAGGCAATTCCTGATGGAAAATGCAAAAGCACATATATGTTTTAATATCTT

        2110              2130              2150
TATGGGCTCTGTTCAAGGCAGTGCTGAGAGGGAGGGGTTATAGCTTCAGGAGGGAACCAG

        2170              2190              2210
CTTCTGATAAACACAATCTGCTAGGAACTTGGGAAAGGAATCAGAGAGCTGCCCTTCAGC
```

2230 2250 2270

GATTATTTAAATTGTTAAAGAATACACAATTTGGGGTATTGGATTTTTCTCCTTTTCTC

2290 2310 2330

TGAGACATTCCACCATTTTAATTTTTGTAACTGCTTATTTATGTGAAAAGGGTTATTTTT

2350 2370 2390

ACTTAGCTTAGCTATGTCAGCCAATCCGATTGCCTTAGGTGAAAGAAACCACCGAAATCC

2410 2430 2450

CTCAGGTCCCTTGGTCAGGAGCCTCTCAAGATTTTTTTGTCAGAGGCTCCAAATAGAAA

2470 2490 2510

ATAAGAAAAGGTTTTCTTCATTCATGGCTAGAGCTAGATTTAACTCAGTTTCTAGGCACC

2530 2550 2570

TCAGACCAATCATCAACTACCATTCTATTCCATGTTTGCACCTGTGCATTTTCTGTTTGC

2590 2610 2630

CCCCATTCACTTTGTCAGGAAACCTTGGCCTCTGCTAAGGTGTATTTGGTCCTTGAGAAG

2650 2670 2690

TGGGAGCACCCTACAGGGACACTATCACTCATGCTGGTGGCATTGTTTACAGCTAGAAAG

2710 2730 2750

CTGCACTGGTGCTAATGCCCCTTGGGAAATGGGGCTGTGAGGAGGAGGATTATAACTTAG

2770 2790 2810

GCCTAGCCTCTTTTAACAGCCTCTGAAATTTATCTTTTCTTCTATGGGGTCTATAAATGT

2830 2850 2870

ATCTTATAATAAAAAGGAAGGACAGGAGGAAGACAGGCAAATGTACTTCTCACCCAGTCT

2890 2910 2930

TCTACACAGATGGAATCTCTTTGGGGCTAAGAGAAAGGTTTTATTCTATATTGCTTACCT

2950 2970 2990

GATCTCATGTTAGGCCTAAGAGGCTTTCTCCAGGAGGATTAGCTTGGAGTTCTCTATACT

3010 3030 3050

CAGGTACCTCTTTCAGGGTTTTCTAACCCTGACACGGACTGTGCATACTTTCCCTCATCC

3070 3090 3110

ATGCTGTGCTGTGTTATTTAATTTTTCCTGGCTAAGATCATGTCTGAATTATGTATGAAA

3130 3150 3170

ATTATTCTATGTTTTTATAATAAAAATAATATATCAGACATCGAAAAAAAAAA

CEA-(f):

```
        10         30         50
         .         .         .         .         .         .
CAGCCGTGCTCGAAGCGTTCCTGGAGCCCAAGCTCTCCTCCACAGGTGAAGACAGGGCCA

        70         90        110
         .         .         .         .         .         .
GCAGGAGACACCATGGGGCACCTCTCAGCCCCACTTCACAGAGTGCGTGTACCCTGGCAG
            MetGlyHisLeuSerAlaProLeuHisArgValArgValProTrpGln

       130        150        170
         .         .         .         .         .         .
GGGCTTCTGCTCACAGCCTCACTTCTAACCTTCTGGAACCCGCCCACCACTGCCCAGCTC
GlyLeuLeuLeuThrAlaSerLeuLeuThrPheTrpAsnProProThrThrAlaGlnLeu

       190        210        230
         .         .         .         .         .         .
ACTACTGAATCCATGCCATTCAATGTTGCAGAGGGGAAGGAGGTTCTTCTCCTTGTCCAC
ThrThrGluSerMetProPheAsnValAlaGluGlyLysGluValLeuLeuLeuValHis

       250        270        290
         .         .         .         .         .         .
AATCTGCCCCAGCAACTTTTTGGCTACAGCTGGTACAAAGGGGAAAGAGTGGATGGCAAC
AsnLeuProGlnGlnLeuPheGlyTyrSerTrpTyrLysGlyGluArgValAspGlyAsn

       310        330        350
         .         .         .         .         .         .
CGTCAAATTGTAGGATATGCAATAGGAACTCAACAAGCTACCCCAGGGCCCGCAAACAGC
ArgGlnIleValGlyTyrAlaIleGlyThrGlnGlnAlaThrProGlyProAlaAsnSer

       370        390        410
         .         .         .         .         .         .
GGTCGAGAGACAATATACCCCAATGCATCCCTGCTGATCCAGAACGTCACCCAGAATGAC
GlyArgGluThrIleTyrProAsnAlaSerLeuLeuIleGlnAsnValThrGlnAsnAsp
```

```
      430                 450                 470
        .         .         .         .         .         .
ACAGGATTCTACACCCTACAAGTCATAAAGTCAGATCTTGTGAATGAAGAAGCAACTGGA
ThrGlyPheTyrThrLeuGlnValIleLysSerAspLeuValAsnGluGluAlaThrGly

      490                 510                 530
        .         .         .         .         .         .
CAGTTCCATGTATACCCGGAGCTGCCCAAGCCCTCCATCTCCAGCAACAACTCCAACCCT
GlnPheHisValTyrProGluLeuProLysProSerIleSerSerAsnAsnSerAsnPro

      550                 570                 590
        .         .         .         .         .         .
GTGGAGGACAAGGATGCTGTGGCCTTCACCTGTGAACCTGAGACTCAGGACACAACCTAC
ValGluAspLysAspAlaValAlaPheThrCysGluProGluThrGlnAspThrThrTyr

      610                 630                 650
        .         .         .         .         .         .
CTGTGGTGGATAAACAATCAGAGCCTCCCGGTCAGTCCCAGGCTGCAGCTGTCCAATGGC
LeuTrpTrpIleAsnAsnGlnSerLeuProValSerProArgLeuGlnLeuSerAsnGly

      670                 690                 710
        .         .         .         .         .         .
AACAGGACCCTCACTCTACTCAGTGTCACAAGGAATGACACAGGACCCTATGAGTGTGAA
AsnArgThrLeuThrLeuLeuSerValThrArgAsnAspThrGlyProTyrGluCysGlu

      730                 750                 770
        .         .         .         .         .         .
ATACAGAACCCAGTGAGTGCGAACCGCAGTGACCCAGTCACCTTGAATGTCACCTATGGC
IleGlnAsnProValSerAlaAsnArgSerAspProValThrLeuAsnValThrTyrGly

      790                 810                 830
        .         .         .         .         .         .
CCGGACACCCCCACCATTTCCCCTTCAGACACCTATTACCGTCCAGGGGCAAACCTCAGC
ProAspThrProThrIleSerProSerAspThrTyrTyrArgProGlyAlaAsnLeuSer
```

```
        850               870               890
         .                 .        .        .        .
CTCTCCTGCTATGCAGCCTCTAACCCACCTGCACAGTACTCCTGGCTTATCAATGGAACA
LeuSerCysTyrAlaAlaSerAsnProProAlaGlnTyrSerTrpLeuIleAsnGlyThr

        910               930               950
         .                 .        .        .        .
TTCCAGCAAAGCACACAAGAGCTCTTTATCCCTAACATCACTGTGAATAATAGTGGATCC
PheGlnGlnSerThrGlnGluLeuPheIleProAsnIleThrValAsnAsnSerGlySer

        970               990              1010
         .                 .        .        .        .
TATACCTGCCACGCCAATAACTCAGTCACTGGCTGCAACAGGACCACAGTCAAGACGATC
TyrThrCysHisAlaAsnAsnSerValThrGlyCysAsnArgThrThrValLysThrIle

       1030              1050              1070
         .                 .        .        .        .
ATAGTCACTGAGCTAAGTCCAGTAGTAGCAAAGCCCCAAATCAAAGCCAGCAAGACCACA
IleValThrGluLeuSerProValValAlaLysProGlnIleLysAlaSerLysThrThr

       1090              1110              1130
         .                 .        .        .        .
GTCACAGGAGATAAGGACTCTGTGAACCTGACCTGCTCCACAAATGACACTGGAATCTCC
ValThrGlyAspLysAspSerValAsnLeuThrCysSerThrAsnAspThrGlyIleSer

       1150              1170              1190
         .                 .        .        .        .
ATCCGTTGGTTCTTCAAAAACCAGAGTCTCCCGTCCTCGGAGAGGATGAAGCTGTCCCAG
IleArgTrpPhePheLysAsnGlnSerLeuProSerSerGluArgMetLysLeuSerGln

       1210              1230              1250
         .                 .        .        .        .
GGCAACACCACCCTCAGCATAAACCCTGTCAAGAGGGAGGATGCTGGGACGTATTGGTGT
GlyAsnThrThrLeuSerIleAsnProValLysArgGluAspAlaGlyThrTyrTrpCys
```

```
       1270                1290                1310
          .         .         .         .         .         .
GAGGTCTTCAACCCAATCAGTAAGAACCAAAGCGACCCCATCATGCTGAACGTAAACTAT
GluValPheAsnProIleSerLysAsnGlnSerAspProIleMetLeuAsnValAsnTyr

       1330                1350                1370
          .         .         .         .         .         .
AATGCTCTACCACAAGAAAATGGCCTCTCACCTGGGGCCATTGCTGGCATTGTGATTGGA
AsnAlaLeuProGlnGluAsnGlyLeuSerProGlyAlaIleAlaGlyIleValIleGly

       1390                1410                1430
          .         .         .         .         .         .
GTAGTGGCCCTGGTTGCTCTGATAGCAGTAGCCCTGGCATGTTTTCTGCATTTCGGGAAG
ValValAlaLeuValAlaLeuIleAlaValAlaLeuAlaCysPheLeuHisPheGlyLys

       1450                1470                1490
          .         .         .         .         .         .
ACCGGCAGCTCAGGACCACTCCAATGACCCACCTAACAAGATGAATGAAGTTACTTATTC
ThrGlySerSerGlyProLeuGln

       1510                1530                1550
          .         .         .         .         .         .
TACCCTGAACTTTGAAGCCCAGCAACCCACACAACCAACTTCAGCCTCCCCATCCCTAAC

       1570                1590                1610
          .         .         .         .         .         .
AGCCACAGAAATAATTTATTCAGAAGTAAAAAAGCAGTAATGAAACCTGAAAAAAAAAAA

       1630
          .
AAAAAAAAAA
```

CEA-(g):

```
        10                  30                  50
CAGCCGTGCTCGAAGCGTTCCTGGAGCCCAAGCTCTCCTCCACAGGTGAAGACAGGGCCA

        70                  90                 110
GCAGGAGACACCATGGGGCACCTCTCAGCCCCACTTCACAGAGTGCGTGTACCCTGGCAG
            MetGlyHisLeuSerAlaProLeuHisArgValArgValProTrpGln

       130                 150                 170
GGGCTTCTGCTCACAGCCTCACTTCTAACCTTCTGGAACCCGCCCACCACTGCCCAGCTC
GlyLeuLeuLeuThrAlaSerLeuLeuThrPheTrpAsnProProThrThrAlaGlnLeu

       190                 210                 230
ACTACTGAATCCATGCCATTCAATGTTGCAGAGGGGAAGGAGGTTCTTCTCCTTGTCCAC
ThrThrGluSerMetProPheAsnValAlaGluGlyLysGluValLeuLeuLeuValHis

       250                 270                 290
AATCTGCCCCAGCAACTTTTTGGCTACAGCTGGTACAAAGGGGAAAGAGTGGATGGCAAC
AsnLeuProGlnGlnLeuPheGlyTyrSerTrpTyrLysGlyGluArgValAspGlyAsn

       310                 330                 350
CGTCAAATTGTAGGATATGCAATAGGAACTCAACAAGCTACCCCAGGGCCCGCAAACAGC
ArgGlnIleValGlyTyrAlaIleGlyThrGlnGlnAlaThrProGlyProAlaAsnSer

       370                 390                 410
GGTCGAGAGACAATATACCCCAATGCATCCCTGCTGATCCAGAACGTCACCCAGAATGAC
GlyArgGluThrIleTyrProAsnAlaSerLeuLeuIleGlnAsnValThrGlnAsnAsp

       430                 450                 470
ACAGGATTCTACACCCTACAAGTCATAAAGTCAGATCTTGTGAATGAAGAAGCAACTGGA
ThrGlyPheTyrThrLeuGlnValIleLysSerAspLeuValAsnGluGluAlaThrGly
```

490 510 530

CAGTTCCATGTATACCCGGAGCTGCCCAAGCCCTCCATCTCCAGCAACAACTCCAACCCT
GlnPheHisValTyrProGluLeuProLysProSerIleSerSerAsnAsnSerAsnPro

550 570 590

GTGGAGGACAAGGATGCTGTGGCCTTCACCTGTGAACCTGAGACTCAGGACACAACCTAC
ValGluAspLysAspAlaValAlaPheThrCysGluProGluThrGlnAspThrThrTyr

610 630 650

CTGTGGTGGATAAACAATCAGAGCCTCCCGGTCAGTCCCAGGCTGCAGCTGTCCAATGGC
LeuTrpTrpIleAsnAsnGlnSerLeuProValSerProArgLeuGlnLeuSerAsnGly

670 690 710

AACAGGACCCTCACTCTACTCAGTGTCACAAGGAATGACACAGGACCCTATGAGTGTGAA
AsnArgThrLeuThrLeuLeuSerValThrArgAsnAspThrGlyProTyrGluCysGlu

730 750 770

ATACAGAACCCAGTGAGTGCGAACCGCAGTGACCCAGTCACCTTGAATGTCACCTATGGC
IleGlnAsnProValSerAlaAsnArgSerAspProValThrLeuAsnValThrTyrGly

790 810 830

CCGGACACCCCCACCATTTCCCCTTCAGACACCTATTACCGTCCAGGGGCAAACCTCAGC
ProAspThrProThrIleSerProSerAspThrTyrTyrArgProGlyAlaAsnLeuSer

850 870 890

CTCTCCTGCTATGCAGCCTCTAACCCACCTGCACAGTACTCCTGGCTTATCAATGGAACA
LeuSerCysTyrAlaAlaSerAsnProProAlaGlnTyrSerTrpLeuIleAsnGlyThr

910 930 950

TTCCAGCAAAGCACACAAGAGCTCTTTATCCCTAACATCACTGTGAATAATAGTGGATCC
PheGlnGlnSerThrGlnGluLeuPheIleProAsnIleThrValAsnAsnSerGlySer

970 990 1010

TATACCTGCCACGCCAATAACTCAGTCACTGGCTGCAACAGGACCACAGTCAAGACGATC
TyrThrCysHisAlaAsnAsnSerValThrGlyCysAsnArgThrThrValLysThrIle

```
            1030                  1050                  1070
               .         .         .         .         .         .
ATAGTCACTGATAATGCTCTACCACAAGAAAATGGCCTCTCACCTGGGGCCATTGCTGGC
IleValThrAspAsnAlaLeuProGlnGluAsnGlyLeuSerProGlyAlaIleAlaGly

            1090                  1110                  1130
               .         .         .         .         .         .
ATTGTGATTGGAGTAGTGGCCCTGGTTGCTCTGATAGCAGTAGCCCTGGCATGTTTTCTG
IleValIleGlyValValAlaLeuValAlaLeuIleAlaValAlaLeuAlaCysPheLeu

            1150                  1170                  1190
               .         .         .         .         .         .
CATTTCGGGAAGACCGGCAGCTCAGGACCACTCCAATGACCCACCTAACAAGATGAATGA
HisPheGlyLysThrGlySerSerGlyProLeuGln

            1210                  1230                  1250
               .         .         .         .         .         .
AGTTACTTATTCTACCCTGAACTTTGAAGCCCAGCAACCCACACAACCAACTTCAGCCTC

            1270                  1290                  1310
               .         .         .         .         .         .
CCCATCCCTAACAGCCACAGAAATAATTTATTCAGAAGTAAAAAAGCAGTAATGAAACCT

            1330
               .
GAAAAAAAAAAAAAAAAAAA
```

The cell lines of the present invention are obtained by gene transfection. Gene transfection is the process by which DNA is introduced into the nucleus of cultured cells and is used with an appropriate selection system to obtain cells which retain and express the transfected DNA as part of their genetic material (F. H. Ruddle, M. E. Kamarck, A. McClelland and L. Kuhn, "DNA Mediated Gene Transfer in Mammalian Gene Cloning", Genetic Engineering, Vol. 6, 319-388 (1984)).

In particular, the preparation of cell lines according to the present invention will involve:

(1) Cotransfection of human DNA, e.g., LoVo cell (ATCC No. CCL 229) DNA with a selectable marker, e.g., the HSV tk gene into recipient cells. The transfection procedure and recipient cells should preferably permit the isolation of several thousand independent transfectants, e.g., the calcium phosphate precipitation procedure using $Ltk^-$ cells as recipients.

(2) Selection of primary transfectant cells expressing a CEA family antigen on their surface, for example, by fluorescence activated cell sorting. Such cells can be detected by antigen specific reagents, e.g., anti-CEA antigen monoclonal or polyclonal antibodies.

(3) Cotransfection of primary transfectant DNA into recipient cells as in (1) above and selection of expressing cells as in (2) above to obtain secondary transfectants.

(4) Selection of transfectants with a higher level of expression by cell sorting of the highest 1% of cells in the secondary population and subcloning from a substantially homogeneous high expression population.

The human DNA used in the primary transfection can be essentially any human DNA from a source that contains the complete human genome, but preferably will be from a source cell line that is known to express a CEA family antigen. In the case of CEA, LoVo cells are known to express CEA.

(5) Alternatively, transfection can occur using a complete cDNA for a CEA family antigen cloned into a cDNA expression vector. This expression vector also may contain a mammalian selectable marker. Nonlimiting examples of expression vectors for use in the present invention include the following: pMV (ATCC No. 37190) and pMVBneo (G.N. Pavalakis et al, Gene Transfer Vectors for Mammalian Cells, Cold Spring Harbor, (1987)).

The transfection procedure into recipient cells and subsequent selection should preferably permit the isolation of several hundred independent transfectants.

(6) Selection of cDNA expressing transfectants as described above in (2).

The recipient non-human mammalian cells likewise can vary widely, provided that they exhibit some properties for selecting transfectants, undergo efficient transfection and do not themselves express the surface protein of interest. Essentially all tissue culture cell lines can be developed to have a selectable marker which can be used to isolate successful transfectants. For example, bacterial neomycin resistance gene (aminoglycoside 3′-phosphotransferase II) which confers resistance to the antibiotic G418 can be used to transfect cells sensitive to G418. While some cell types may be inefficient recipients using the conventional calcium phosphate method, alternative methods such as electroporation or protoplast fusion are available. Provided that several thousand independent transfectants can be achieved, any cell type can be used in the role of the recipient. In the case of the human antigens, some human cell types and similar cells cannot be used because they normally express the desired cell surface protein.

$Ltk^-$ cells (NCTC clone 929; ATCC No. CCL1) derived from a murine tumor are particularly useful as recipient cells. They are additionally selected to be deficient in thymidine kinase activity due to a non-reverting mutation in the cellular thymidine kinase gene. HAT media provides a powerful section for tk activity which can readily be restored to $Ltk^-$ (CCL1 $tk^-$) cells by transfection of the Herpes Simplex Virus Thymidine kinase (tk) gene. Thus a well characterized, efficient and convenient selection for cells which have become stably transfected is afforded by the use of these cells. $Ltk^-$ cells are among the most efficient recipient cells for the calcium phosphate transfection procedure. Frequencies as high as 1 cell in 300 can be obtained. Since at least several thousand independent transfectants are required for successful genomic transfection of a gene, it is preferable to use recipient cells which transfect efficiently. $Ltk^-$ cells do not express any of the CEA family antigens. Since the selection of transfectants involves an indirect immunofluorescence assay, this can be an essential property of the recipient cells. The repetitive DNA sequences in $Ltk^-$ cells do not cross hybridize with human repetitive sequences. Human DNA in $Ltk^-$ cells can therefore be detected by repetitive sequence probes. Since an objective of generating the transfectants is the molecular cloning of the gene, it is important that the recipient cells by of a species other than human which meets this criterion.

Genomic primary, genomic secondary and cDNA transfectants can be used as immunogens to produce antibodies, particularly monoclonal antibodies, to CEA family members. The transfectants provide a more specific immunogen than, for example, LoVo colon carcinoma cells, because the human tumor line expresses more than one of the CEA family antigens. Also the species of the mammalian cells expressing the human surface protein and the species of the immunized animal can be the same, e.g., mouse, yielding an immunogenic response directed specifically only to the foreign protein. Antibodies produced in this manner and antibodies produced by any other method will be selected for the ability to bind to a specific CEA family antigen by a number of methods, e.g., indirect immunofluorescence and FACS analysis. This will serve to delineate specificity of each antibody for CEA family antigens and will provide key information leading to identification of candidate antibodies for diagnostic and therapeutic approaches.

The present invention also provides a method for producing monoclonal antibodies to CEA family antigens by somatic cell hybridization techniques. In general, a monoclonal antibody of the present invention is produced by immunizing a competent host animal with either non-human mammalian cells which express CEA family antigens (prepared by gene transfection as described above), or purified CEA family antigens from such cell lines (e.g., by subjecting the cells of the invention to affinity chromatography), fusing lymphocytes from such host animal which produce antibody to CEA family antigens with myeloma cells to form hybridomas, isolating a hybridoma clone which secretes the desired antibody and harvesting the secreted monoclonal antibody. The lymphocytes involved in the hybridization are commonly spleen cells removed from an immunized animal such as a mouse or rat. Preferably, both the lymphocytes and myeloma cells are of murine origin, with murine myeloma cells deficient in the enzyme hypoxanthine guanine phosphoribosyl transferase being particularly useful. For reviews and commentary on the production of monoclonal antibodies by somatic cell hybridization, reference may be made to the following: Lymphocyte Hybridomas, ed. Melchers et al, Springer-Verlag (New York 1978); Nature, 266 495 (1977); Med. Lab. Sci., 36, 329 (1979); and Science, 208, 692 (1980).

Formed hybridomas which secrete the desired antibody are isolated, usually by cloning on culture media selective for the fused cell hybridomas and determining by appropriate methods an isolated hybridoma which secretes the desired antibody. The homogeneity of the hybridoma cell line is thereafter preferably assured by subcloning the hybridomas selected for their desirable antibody secretion. Harvesting of the secreted monoclonal antibodies is performed by conventional techniques. Proliferation of antibodies can be accomplished by culturing hybridomas in vitro or in vivo, e.g., introducing the hybridoma into an animal such as a mouse and removing antibody-rich ascites fluid.

Lymphocytes producing antibody to CEA family antigens can be obtained from various sites, usually the lymph nodes or spleen. Usually, the selected lymphocytes are spleen cells from the immunized animal. Immunization can be accomplished by injection of the host animal, e.g., mouse, rat, or goat, at one or more of a variety of sites with the receptor-expressive non-human mammalian cells, preferably high expressing cells. Further injections are made at the same or different sites at regular or irregular intervals thereafter until it is evident that anti-receptor antibodies are being produced in vivo.

Myeloma cells, that is, malignant cells from primary bone marrow tumors, produce immunoglobulins with unknown specificity and have the ability to propagate in vitro. In contrast, individual lymphocytes characterized by the secretion of immunoglobulins with known specificity, e.g., antibodies capable of binding a known antigen or hapten, are labile under in vitro culturing. A hybridoma, i.e., a hybrid cell formed by fusion of a single lymphocyte with a single myeloma cell, retains the desired immunological specificity of the lymphocyte parent cell and the desired in vitro stability of the myeloma parent cell. Myeloma cells of various animal origin can be used according to the present invention, for example, myeloma cells from mice or rats, however, for reasons of genetic stability it is preferred to fuse lymphocytes and myeloma cells derived from the same animal species and most preferably, from the same strain of such animal species. Murine lymphocytes and myeloma cells are most commonly use, particularly from the BALB/c strain.

Myeloma cells may be of the secreting or nonsecreting type, the former characterized by secretion of immunoglobulin or fragments thereof from the cell into the surrounding medium. Nonsecreting cell lines are preferred since the resulting hybridomas will feature secretion of only the specific immunoglobulin (e.g., IgG, IgE, IgM, IgA or IgD classes) produced by the parent lymphocyte. Moreover, myeloma cells which have been rendered deficient in a particular nucleotide synthetase enzyme are particularly preferred since such a circumstance provides ready means for isolating hybridomas from nonfused lymphocytes and myeloma cells. When grown on a culture medium containing the substrate or substrates for the nucleotide synthetase enzyme in which the myeloma cell is deficient as the only source of nucleotide formation, hybridomas will survive and proliferate since the deficient enzyme will be provided by retained genetic features from the lymphocyte. However, nonfused myeloma cells will die because of enzyme deficiency and nonfused lymphocytes will die due to their inherent in vitro liability. Preferred myeloma cell lines are those deficient in the enzyme hypoxanthine guanine phosphoribosyl transferase (HGPRT) and unable to survive on culture media containing hypoxanthine, aminopterin and thymidine for nucleotide synthesis (such media being conventionally referred to as HAT media -Science, 145, 709 (1964). Particular myeloma cell lines which are useful in the present method are conventionally known in the art.

Fusing of the lymphocyte and myeloma cells to form hybridomas is accomplished by conventional means, usually by mixing the cells in the presence of a fusing agent such as sendai virus or polyethylene glycol (PEG). PEG is the preferred fusing agent and is available in commercial lots which vary widely in degree of purity and suitability for in vitro application. Optimal molecular weights for PEG fusing agents have not been established, with molecular weights between about 1000 and 4000 (commercially available as PEG 1000, etc.) being conventionally used.

The mechanism of fusion is generally unknown and yields efficiencies of one hybridoma formed for about every $10^4$ normal cells present. The ratio of the number of lymphocytes to myeloma cells is usually greater than one, more usually 5 or more. Since an unscreened population of lymphocytes is made available to the myeloma cells for fusion, resulting hybridomas will express a distribution of varying antibody secretions. A hybridoma clone, i.e., a population of cells having a common single cell origin, which secretes the desired antibody is isolated by conventional techniques. A common technique involves the division of the fusion mixture into a multiplicity of diluted volumes in the expectation that the majority of resulting volumes which contain hybridomas will contain no more than one. Preferably, the individual volumes will comprise a selective culture medium, e.g., the HAT medium mentioned above, in which only hybridomas will survive. Aliquots of the separate culture fluids are removed after an appropriate incubation period and subjected to an assay for the desired antibodies. Many methods are commonly used in this screening step, including immunoassay procedures such as radioimmunoassay or non-isotopic immunoassays of the homogenous or heterogeneous type. Culture fluids which are positive for the desired antibody are then usually subcloned, such as by further limiting dilution steps, to assure monoclonal isolation and for stability of the desired hybridoma.

The desired monoclonal antibodies can be harvested by several different methods. In one method, the hybridoma clone is cultured in vitro for an appropriate length of time and aliquots of the culture fluid drawn off to provide monoclonal antibody-rich fractions. Alternatively, the hybridoma clone is injected into or implanted in a host animal, usually a mouse, resulting in in vitro tumor growth and accumulation of large amounts of monoclonal antibody in the ascites fluid which can be appropriately tapped to permit removal of antibody-rich fractions.

The cell lines of the present invention can also be used to screen monoclonal antibodies or polyclonal antibodies for their specificity in recognizing different members of the CEA antigen family. For example, cell lines can be incubated with antibodies and the antibody binding to such cells be detected by conventional immunological methods such as by, for example, RIA, ELISA and indirect immunofluorescence.

The present invention will now be illustrated, but is not intended to be limited, by the following examples.

## EXAMPLES

### Example 1: Gene Transfection

The gene transfection method involves the introduction and stable integration of either human genomic DNA into mouse Ltk$^-$ cells (thymidine kinase deficient mouse L cells) by calcium phosphate co-precipitation with the Herpes Simplex Virus thymidine kinase (HSVtk) gene or the introduction and stable integration of cloned cDNA into a eucaryotic expression vector containing a selectable marker into Ltk$^-$ cells by calcium phosphate precipitation. Transfected cells were stained with a fluorescent probe specific for the surface molecule of interest and positive cells were enriched from the total population using a Fluorescence Activated Cell Sorter (FACS).

Specifically, mouse Ltk$^-$ cells were co-transfected with genomic DNA and the HSV tk gene using the standard calcium phosphate procedure and HAT (Graham and Van der Eb, Virology, 52, 456-467, (1973)).

### Example 2: Detection of Cells Expressing CEA Family Members

In order to detect cells expressing the isolated CEA family members, an indirect immunofluorescence assay was used. 1-5 x $10^6$ cells were removed from tissue culture using a phosphate buffered salt solution containing the divalent cation chelator ethylenediamine tetraacetic acid (EDTA). These cells were then reacted with saturating quantities of rabbit polyclonal or mouse monoclonal antibodies recognizing CEA antigens for 30 to 60 minutes at 4°C. The cells were sequentially incubated with fluoresceinated goat or rabbit anti-mouse IgG at 4°C for 30 to 60 minutes. Sterile cell sorting was performed using a Becton Dickinson FACS IV, with sorting parameters which selected the brightest cells. Sorted cells were expanded and re-sorted until analysis showed at least 95% positive cells. Single cell cloning was used to establish clonal cell lines which were shown to be 100% positive for expression of the surface antigen.

### Example 3: Isolation of Mouse L-Cell Transfectants with Genomic Genes

The isolation of mouse L-cell transfectants was initiated using indirect immunofluorescence as described above. For the transfection of the genomic gene a pool of L-cells transfected with LoVo DNA and representing 20,000 independent cell clones was subjected to preparative cell sorting. After repeated rounds of cell sorting, 100% of the cells were positive, showing specific fluorescence in the immunofluorescence assay. Single cell cloning was used to isolate cloned cell lines which represented either high (ZH) or (ZL) levels of specific immunofluorescence. Positive cells from these clones were recovered and identified as ZL1 and ZH2. DNA was extracted from each of the lines and analyzed by Southern blotting and hybridization to a HeLa DNA probe. This example confirmed that each of the cell lines contained human DNA, and that the two independent clones represented derivatives from a single parental colony. As expected for primary transfectants, each cell line contained a large amount of human DNA, estimated to be several hundreds of kilobases, based on hybridization intensities when probed with a human repetitive sequence probe.

To obtain secondary transfectants, DNA from a primary transfectant cell line was co-transfected into Ltk$^-$ cells and cell sorting was performed as described above. A total of four clonal secondary cell lines designated as 23.15, 23.25, 23.32 and 23.44 were obtained. A high expressing cell line was isolated by repeated rounds of sorting of the secondary pool until a homogenous level of expression was obtained, followed by subcloning. This line, designated as 23.411 (deposited with the American Type Culture Collection (ATCC) on June 1, 1988, deposit no. CRL 9731, was found to express approximately 20 fold the level of surface immunofluorescence as the other cells clones described based on immunofluorescence intensity. These genomic transfectants contain and express the genes for CEA- (c) (TM-1 CEA), CEA-(e) (TM-2 CEA), CEA-(f) (TM-3 CEA) and CEA-(g) (TM-4 CEA), the transmembrane CEAs.

Example 4: Isolation of Mouse Cells Transfected with Complete cDNAs in an Expression Vector

The isolation of mouse L-cell transfectants expressing cDNA's coding for each of CEA-(b), CEA-(c), CEA-(d) and CEA-(e) was initiated using the indirect immunofluorescence binding assay described above. A pool of L-cells transfected with each cDNA in expression vector MTI-1 derived from pMVBneo (pMVBneo was derived from pSV2neo, ATCC No.37149) and representing 1000 to 5000 independent transfection events was subjected to cell sorting analysis. L-cells expressing CEA-(c) and CEA-(e) expressed the transmembrane CEAs on 100% of the transfected cells and were subcloned. This produced cell line 16.6 (ATCC No. CRL 9735, deposited with the American Type Culture Collection (ATCC) on June 2, 1988), which expresses the cDNA for CEA-(c) or TM-1 CEA, and cell line 22.7, ATCC No. 9730, deposited with the ATCC on June 1, 1988, which expresses the cDNA for CEA-(e) or the alternately spliced TM-2 CEA. L-cells transfected with CEA-(b) and CEA-(d) in the expression vector displayed detectable surface antigen on 1 to 2% of the cells. These antigen positive cells were selected by FACS sorting, and subcloned. This produced cell lines 1LV7-1.2 (ATCC No. 9733, deposited with the ATCC on June 1, 1988), which expresses the cDNA for CEA-(b) or CEA and cell line BT-20.4, ATCC No. 9732, deposited with the ATCC on June 1, 1988, which expresses the cDNA for CEA-(d) or NCA.

In an identical fashion, cell lines epxressing the cDNA's for CEA-(f) and CEA-(g) are produced from the complete cDNA's coding for these proteins.

Example 5: Construction of a Vector for Expression of a cDNA in Eucaryotic Cells

Restriction endonucleases are used to digest a eucaryotic expression vector in a region downstream (3′) from the eucaryotic promoter. In this example the expression vector pMTI-1 (which is derived from pMVBneo with a unique EcoRI site introduced 3′ to the metallothionein promoter) containing the mouse metallothionein is digested with the restriction endonuclease EcoRI. A complete cDNA for a member of the CEA gene family in this example, CEA-(b), is also isolated by restriction endonuclease digestion with EcoRI. The insert is ligated into the expression vector and resultant ligation mixture is used to transform HB101 (ATCC No. 33694) bacteria. Alkaline minilysate preparations of transformed bacteria are analyzed by EcoRI digestion to detect the presence of the cDNA insert and with Kpn I and Sac I to determine the correct orientation of the cDNA in the expression vector. Plasmid DNA with the appropriate insert orientation are used for eucaryotic cell transfection.

Example 6: Screening Antibodies for Specificity Using Transfected Cell Lines

A panel of monoclonal antibodies which react with CEA antigen family members are tested by indirect immunofluorescence for reactivity with cell lines 1LV7-1.2 and 23.411 and BT-20.4 and a panel of antibodies including commercially available antibodies directed to CEA, namely DAKO (Cat No. 4115, Dako Corp., Santa Barbara, CA., U.S.A.), ZYMED (Cat. No. 03-6551, Zymed Laboratories, San Francisco, CA., U.S.A.) and Hybritech (Cat. No. 0062, Hybritech Incorporated, La Jolla, CA, U.S.A.). They are used in saturating quantities to label cell lines by the immunofluorescence assay described herein. Immunofluorescence analysis by the FACS indicated that all three of the above antibodies reacted with these three cell lines. This indicated the lack of specificity of these antibodies for individual members of the CEA antigen family.

Example 7: Production of Antibodies Using These Cell Lines

Transfectant cell line 23.411 was used to immunize C3H/HeJ x Balb/cJ mice. 1 x $10^7$ live cells were

injected intraperitoneally into these mice biweekly for two months. The spleens of four of these mice were removed three days following the last cell injection and 615 hybridomas were produced following fusion with hybridoma parent line P3X63-Ag8.643 (ATCC No. CRL 1580). Supernatants from these hybridomas were assayed by immunofluorescence for their reactivity with cell line 23.411. Hybridoma supernatants positive for 23.411 were subsequently tested for their reactivity with the L cell parent and hybridomas producing antibodies to L cells were discarded. Hybridomas producing supernatant antibodies directed to cell line 23.411 were subcloned and retested for their reactivity. A hybridoma designated CII109.9aC3 was produced which demonstrated reactivity for 23.411 and not for the L cell parent.

It will be appreciated that the instant specification and claims are set forth by way of illustration and not limitation and that various modifications and changes may be made without departing from the spirit and scope of the present invention.

## Claims

1. A non-human mammalian cell line charaterized in that the cell line expresses CEA family cell surface antigens.

2. A cell line according to claim 1 characterized in that it is a primary transfectant prepared by gene transfection of human DNA into non-human mammalian cells.

3. A cell line according to claim 2, wherein the human DNA is LoVo cell genomic DNA and the non-human mammalian cells are mouse $Ltk^-$ cells.

4. A cell line according to claim 1, characterized in that it is a secondary transfectant prepared by gene transfection of primary transfectant DNA into non-human mammalian cells.

5. A cell line according to claim 4, wherein the primary transfectant DNA is from a transfectant prepared by gene transfection of LoVo cell genomic DNA into mouse $Ltk^-$ cells and wherein the non-human mammalian cells of the secondary transfectant are also mouse $Ltk^-$ cells.

6. A cell line according to claim 4, characterized in that it is a substantially homogeneous high expression cloned cell line derived from secondary transfectants, which cloned cell line expresses about twenty fold or greater more human CEA family antigen than secondary transfectants.

7. A cell line according to claim 1, which is prepared by the transfection of a cDNA coding a CEA family antigen which has been cloned into a cDNA expression vector in non-human mammalian cells, preferably wherein said cDNA is selected from the group consisting of CEA-(b), CEA-(c), CEA-(d), CEA-(e), CEA-(f) and CEA-(g).

8. A cell line according to claim 7, designated as CRL 9731, CRL 9730, CRL 9733, or CRL 9732.

9. A cell line according to claims 1-8, wherein said cell line is a mouse cell line.

10. A monoclonal antibody to a CEA family antigen, said monoclonal antibody prepared by a method comprising the steps of (A) immunizing a competent host animal with (i) non-human mammalian cells from a cell line according to any of claims 1-9 which express said antigen or (ii) said antigen purified from said cells, (B) fusing lymphocytes isolated from the host animal which produce antibodies to said receptor with myeloma cells to form hybridomas, (C) isolating a hybridoma clone which secretes a monoclonal antibody to said antigen, and (D) harvesting the secreted monoclonal antibody.